# NOUVEAU FASCICULE DE BREVET EUROPEEN

(11) **EP 0 852 135 B2**
(45) Date de publication et mention de la décision concernant l'opposition: **19.03.2008**
(45) Mention de la délivrance du brevet: 28.04.1999
(21) Numéro de dépôt: 97402864.9
(22) Date de dépôt: 27.11.1997
(51) Int. Cl.: A61K 8/41, A61Q 5/10

(54) **Composition de teinture d'oxydation des fibres kératiniques et procédé de teinture mettant en oeuvre cette composition**
Oxidationsfärbemittel für keratinische Fasern und Färbeverfahren mit diesem Mittel
Oxidative dyeing composition for keratin fibres and dyeing method employing it

(30) Priorité: 23.12.1996 FR 9615894
(43) Date de publication de la demande: 08.07.1998
(73) Titulaire: L'ORÉAL, 75008 Paris (FR)
(72) Inventeur: Rondeau, Christine, 78500 Sartrouville (FR); Cotteret, Jean, 78480 Verneuil Sur Seine (FR); De La Mettrie, Roland, 78110 Le Vesinet (FR)
(74) Mandataire: Dossmann, Gérard

(56) Documents cités:
- EP-A- 0 714 954
- EP-A- 0 739 622
- WO-A-95/01772
- WO-A-95/15144
- WO-A-97/39727
- DE-A- 2 543 100
- FR-A- 2 615 732
- GB-A- 1 211 801
- GB-A- 2 180 215

## Description

La présente invention a pour objet une composition prête à l'emploi pour la teinture d'oxydation des fibres kératiniques, en particulier des fibres kératiniques humaines telles que les cheveux, comprenant, dans un milieu approprié pour la teinture, au moins une base d'oxydation choisie parmi les paraphénylènediamines et les bis-phénylalkylènediamines, en association avec au moins un coupleur choisi parmi des méta-phénylènediamines, au moins un colorant direct cationique sélectionné et au moins un agent oxydant, ainsi que le procédé de teinture mettant en oeuvre cette composition. Elle concerne également un kit de coloration pour la préparation d'une telle composition prête à l'emploi.

Il est connu de teindre les fibres kératiniques et en particulier les cheveux humains avec des compositions tinctoriales contenant des précurseurs de colorant d'oxydation, en particulier des ortho ou paraphénylènediamines, des ortho ou paraaminophénols, appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques.

La variété des molécules mises en jeu au niveau des bases d'oxydation et des coupleurs, permet l'obtention d'une riche palette de couleurs.

Il est également connu que pour faire encore varier les nuances obtenues et leur donner des reflets, on peut utiliser, en association avec les précurseurs de colorants d'oxydation et les coupleurs, des colorants directs, c'est à dire des substances colorées qui apportent une coloration en l'absence d'agent oxydant (voir EP-A-0 739 622).

La coloration dite "permanente" obtenue grâce à ces colorants d'oxydation, doit par ailleurs satisfaire un certain nombre d'exigences. Ainsi, elle doit permettre d'obtenir des nuances dans l'intensité souhaitée et présenter une bonne tenue face aux agents extérieurs (lumière, intempéries, lavage, ondulation permanente, transpiration, frottements).

Les colorants directs appartiennent pour leur très grande majorité à la famille des composés nitrés de la série benzénique et ont l'inconvénient, lorsqu'ils sont incorporés dans des compositions tinctoriales, de conduire à des colorations présentant une ténacité insuffisante, en particulier vis-à-vis des shampooings.

La présente invention vise à proposer de nouvelles compositions pour la coloration d'oxydation des fibres kératiniques et en particulier des fibres kératiniques humaines telles que les cheveux qui permettent d'aboutir à des colorations riches en reflets tout en présentant de bonne propriétés de ténacité, en particulier

Ainsi, la demanderesse vient en effet de découvrir qu'il est possible d'obtenir de nouvelles teintures à la fois lumineuses et tenaces en associant :
- au moins une base d'oxydation choisie parmi les paraphénylènediamines, les bis-phénylalkylènediamines, et leur sels d'addition avec un acide,
- au moins un coupleur choisi parmi les méta-phénylènediamines, et leurs sels d'addition avec un acide,
- au moins un colorant direct cationique de formule (I) ci-après, et
- au moins un agent oxydant

L'invention a donc pour premier objet une composition prête à l'emploi pour la teinture d'oxydation des fibres kératiniques et en particulier des fibres kératiniques humaines telles que les cheveux, caractérisée par le fait qu'elle comprend, dans un milieu approprié pour la teinture :
- au moins une base d'oxydation choisie parmi les paraphénylènediamines, les bis-phénylalkylènediamines, et leur sels d'addition avec un acide.
- au moins un coupleur choisi parmi les méta-phénylènediamines, et leurs sels d'addition avec un acide,
- au moins un colorant direct cationique choisi parmi les composés de formule (I) suivante : 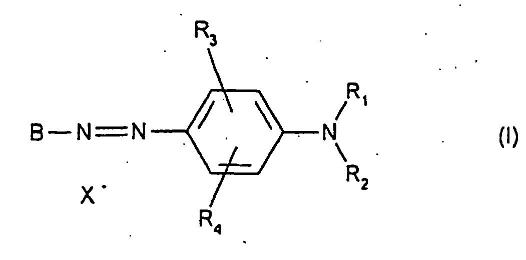
dans laquelle:
R₁ représente un atome d'hydrogène ou un radical alkyle en C₁-C₄,
R₂ représente un atome d'hydrogène, un radical alkyle pouvant être substitué par un radical -CN ou par un groupement amino, un radical 4'-aminophényle ou forme avec R₁ ou avec un atome de carbone du noyau benzénique supportant les radicaux R₃ et R₄, un hétérocycle éventuellement oxygéné et/ou azoté pouvant être substitué par un radical alkyle en C₁-C₄,
R₃ et R₄, identiques ou différents, représentent un atome d'hydrogène, un atome d'halogène tel que le brome, le chlore, l'iode ou le fluor, un radical alkyle en C₁-C₄ ou alcoxy en C₁-C₄, un radical -CN,
X⁻ représente un anion de préférence choisi parmi le chlorure, le méthyl sulfate et l'acétate,
B représente un groupement choisi par les structures B1 à B11 suivantes : 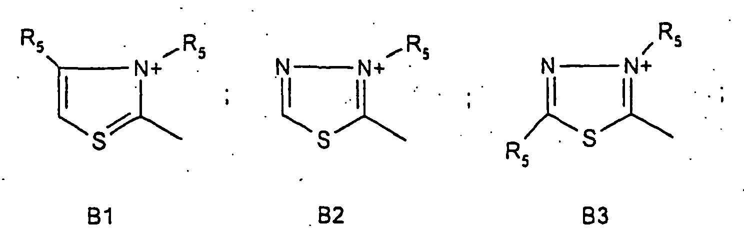 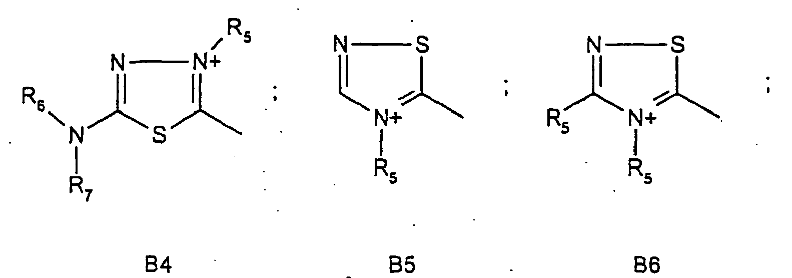 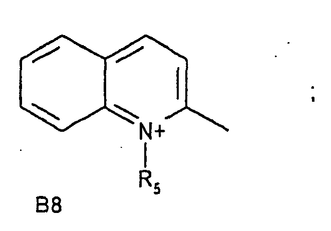 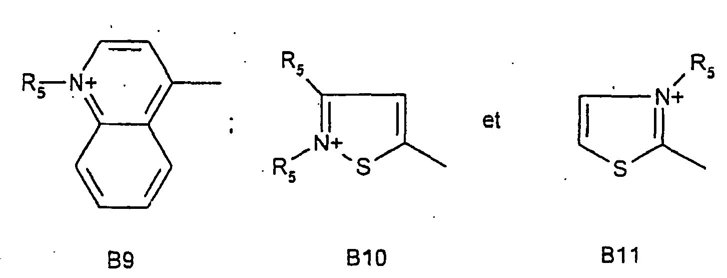
dans lesquelles R₅ représente un radical alkyle en C₁-C₄, R₆ et R₇, identiques ou différents, représentent un atome d'hydrogène ou un radical alkyle en C₁-C₄ ; lorsque R₁ et R₂ forment un hétérocycle azoté, ou lorsque R₃ et R₄ représentent un radical alkyle en C₁-C₄ ou un radical alcoxy en C₁-C₄, ou lorsque R₂ représente un radical 4'-aminophényle, alors B peut également représenter un groupement de structure B12 suivante : 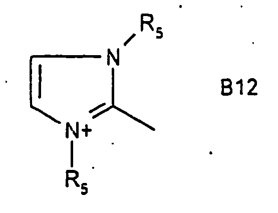 dans laquelle R₅ a la même signification que celle indiquée ci-dessus pour les structures B1 à B11; et
- au moins un agent oxydant.

Les compositions tinctoriales prêtes à l'emploi conformes à l'invention permettent d'aboutir à des colorations dans des nuances naturelles ou cendrées présentant une bonne résistance aux différents traitements que peuvent subir les cheveux et en particulier vis-à-vis des shampooings.

L'invention a également pour objet un procédé de teinture d'oxydation des fibres kératiniques mettant en oeuvre cette composition tinctoriale prête à l'emploi.

Les paraphénylènediamines utilisables à titre de base d'oxydation dans les compositions tinctoriales prêtes à l'emploi conformes à l'invention sont de préférence choisies parmi les composés de formule (II) suivante, et leur sels d'addition avec un acide : 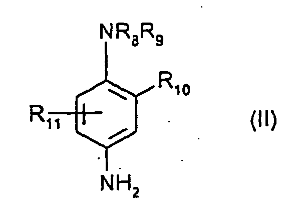 dans laquelle :
R₈ représente un atome d'hydrogène, un radical alkyle en C₁-C₄, monohydroxyalkyle en C₁-C₄, polyhydroxyalkyle en C₂-C₄, phényle, 4'-aminophényle ou alcoxy(C₁-C₄)alkyle en C₁-C₄,
R₉ représente un atome d'hydrogène, un radical alkyle en C₁-C₄, monohydroxyalkyle en C₁-C₄ ou polyhydroxyalkyle en C₂-C₄,
R₁₀ représente un atome d'hydrogène, un atome d'halogène tel qu'un atome de chlore, de brome, d'iode ou de fluor, un radical alkyle en C₁-C₄, monohydroxyalkyle en C₁-C₄, hydroxyalcoxy en C₁-C₄, mésylaminoalcoxy en C₁-C₄, carbamoylaminoalcoxy en C₁-C₄ ou acétylaminoalcoxy en C₁-C₄.
R₁₁ représente un atome d'hydrogène ou un radical alkyle en C₁-C₄.

Parmi les paraphénylènediamines de formule (II) ci-dessus, on peut plus particulièrement citer la paraphénylènediamine, la paratoluylènediamine, la 2-chloro paraphénylènediamine, la 2.3-diméthyl paraphénylènediamine, la 2,6-diméthyl paraphénylènediamine, la 2,6-diéthyl paraphénylènediamine, la 2,5-diméthyl paraphénylènediamine, la N.N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-amino N,N-bis-(β-hydroxyéthyl) 3-méthyl aniline, la 4-amino 3-chloro N,N-bis-(β-hydroxyéthyl) aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, et leurs sels d'addition avec un acide.

Parmi les paraphénylènediamines de formule (II) ci-dessus, on préfère tout particulièrement la paraphénylènediamine, la paratoluylènediamine, la 2-isopropyl paraphénylènediamine, la 2-β-hydroxyéthyl paraphénylènediamine, la 2-β-hydroxyéthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, et leurs sels d'addition avec un acide.

Les bis-phénylalkylènediamines utilisables à titre de base d'oxydation dans les compositions tinctoriales prêtes à remploi conformes à l'invention sont de préférence choisies parmi les composés de formule (III) suivante, et leurs sels d'addition avec un acide : 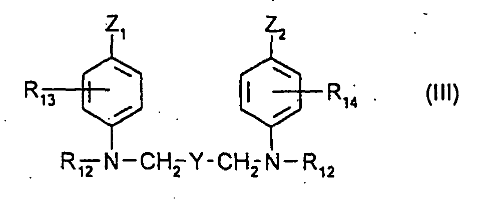 dans laquelle :
Z₁ et Z₂, identiques ou différents, représentent un radical hydroxyle ou NHR₁₅ dans lequel R₁₅ représente un atome d'hydrogène ou un radical alkyle en C₁-C₄,
R₁₂ représente un atome d'hydrogène, un radical alkyle en C₁-C₄, monohydroxyalkyle en C₁-C₄- polyhydroxyalkyle en C₂-C₄ ou aminoalkyle en C₁-C₄ dont le reste amino peut être substitué,
R₁₃ et R₁₄, identiques ou différents, représentent un atome d'hydrogène ou d'halogène ou un radical alkyle en C₁-C₄.
Y représente un radical pris dans le groupe constitué par les radicaux suivants : -(CH₂)ₙ ; -(CH₂)ₘ-O-(CH₂)ₘ ; - (CH₂)ₘ-CHOH-(CH₂)ₘ et 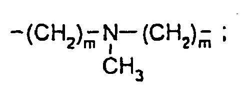
dans lesquels n est un nombre entier compris entre 0 et 8 inclusivement et m est un nombre entier compris entre 0 et 4 inclusivement.

Parmi les bis-phénylalkylènediamines de formule (III) ci-dessus, on peut plus particulièrement citer le N,N'-bis-(β-hydroxyéthyl) N,N'-bis-(4'-aminophény) 1.3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthylaminophényl) tétraméthylènediamine, la N,N'-bis-(éthyl) N,N'-bis-(4'-amino, 3'-méthylphényl) éthylènediamine, et leurs sels d'addition avec un acide.

Parmi ces bis-phénylalkylènediamines de formule (III), le N,N'-bis-(β-hydroxyéthyl) N,N'-bis-(4'-aminophényl) 1,3-diamino propanol ou l'un de ses sels d'addition avec un acide sont particulièrement préférés.

Les méta-phénylènediamines utilisables à titre de coupleur dans les compositions tinctoriales prêtes à l'emploi conformes à l'invention sont de préférence choisies parmi les composés de formule (IV) suivante, et leurs sels d'addition avec un acide : 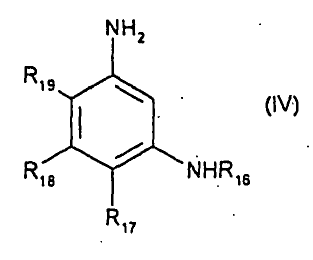 dans laquelle:
- R₁₆ représente un atome d'hydrogène, un radical alkyle en C₁-C₄, monohydroxyalkyle en C₁-C₄ ou polyhydroxyalkyle en C₂-C₄ ;
- R₁₇ et R₁₈, identiques ou différents, représentent un atome d'hydrogène, un radical alkyle en C₁-C₄, monohydroxyalcoxy en C₁-C₄ ou polyhydroxyalcoxy en C₂-C₄ ;
- R₁₉ représente un atome d'hydrogène, un radical alkoxy en C₁-C₄, aminoalkoxy en C₁-C₄, monohydroxyalkoxy en C₁-C₄, polyhydroxyalkoxy en C₂-C₄ ou un radical 2,4-diaminophénoxyathoxy.

Parmi les méta-phénylènediamines de formule (IV) ci-dessus, on peut plus particulièrement citer la métaphénylènediamine, le 3,5-diamino 1-éthyl 2-méthoxybenzène, le 3,5-diamino 2-méthoxy 1-méthyl benzène, le 2,4-diamino 1-éthoxybenzène, le 1,3-bis-(2,4-diaminophénoxy) propane, le bis-(2,4-diaminophénoxy) méthane, le 1-(β-aminoéthyloxy) 2,4-diamino benzène, le 2-amino 1-(β-hydroxyéthyloxy) 4-méthylamino benzène, le 2,4-diamino 1-éthoxy 5-méthyl benzène, le 2,4-diamino 5-(β-hydroxyéthyloxy) 1-methylbenzène, le 2,4-diamino 1-(β,γ-dihydroxypropyloxy) benzène, le 2,4-diamino 1-(β-hydroxyéthyloxy) benzène, le 2-amino 4-N-(β-hydroxyéthyl) amino 1-méthoxy benzène, et leurs sels d'addition avec un acide.

Les sels d'addition avec un acide utilisables dans le cadre des compositions tinctoriales de l'invention sont notamment choisis parmi les chlorhydrates, les bromhydrates, les sulfates et les tartrates.

Les colorants directs cationiques de formule (1) utilisables dans les compositions tinctoriales prêtes à l'emploi conformes à l'invention, sont des composés connus et sont décrits par exemple dans les demandes de brevets WO 95/01772, WO 95115144 et EP-A-0 714 954.

Parmi les colorants directs cationiques de formule (1) utilisables dans les compositions tinctoriales prêtes à l'emploi conformes à l'invention, on peut plus particulièrement citer les composés répondant aux structures (I1) à (126) suivantes : 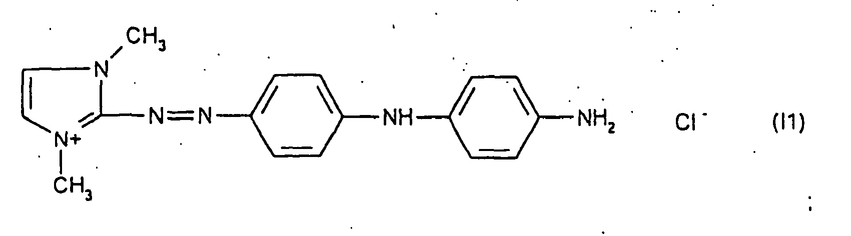 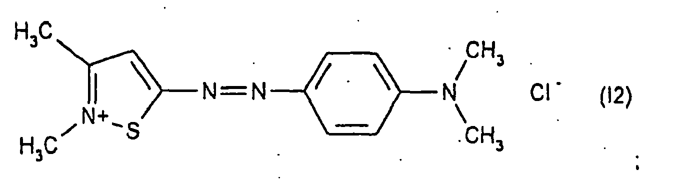 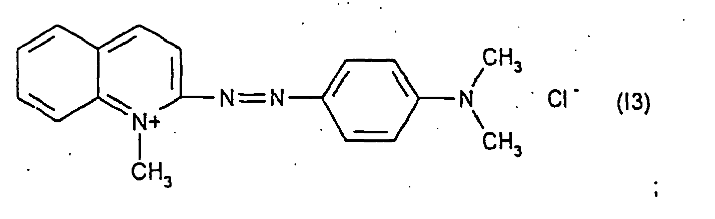 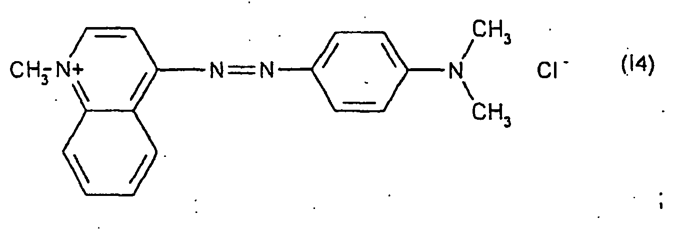 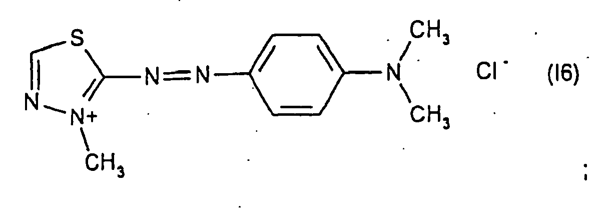 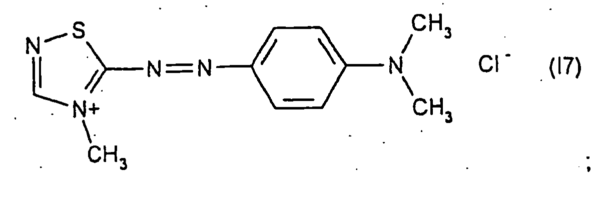 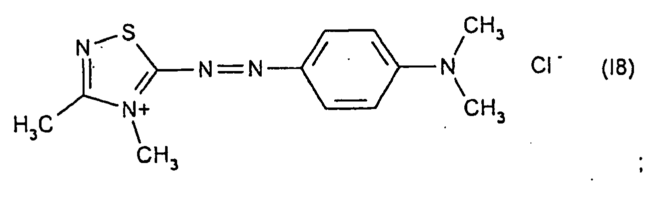 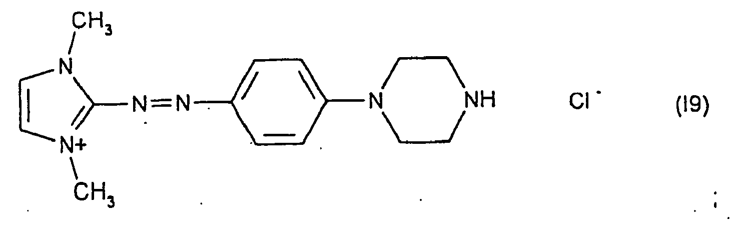 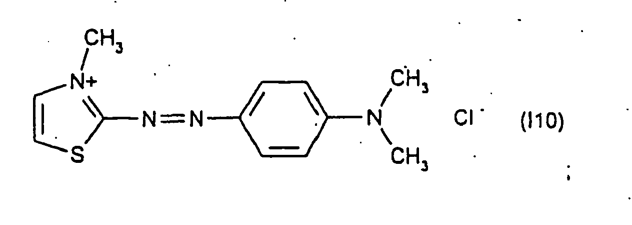 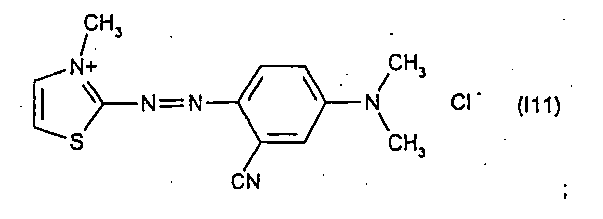 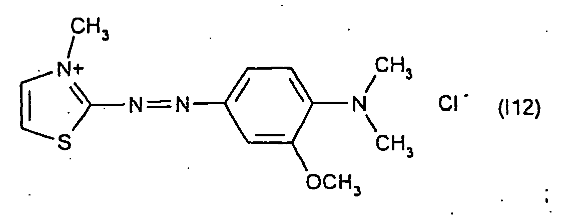 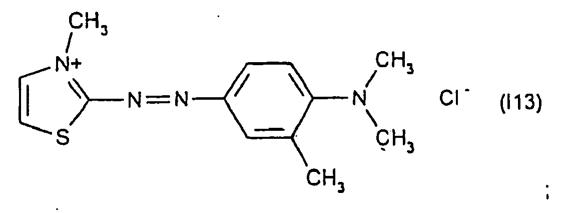 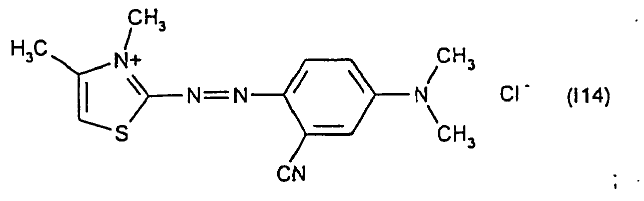 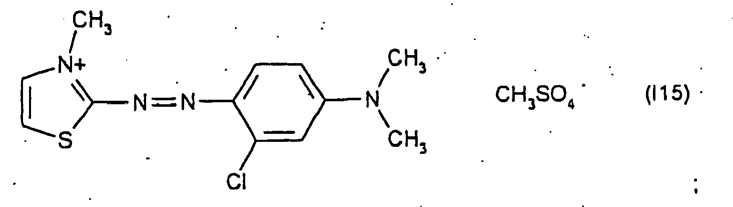 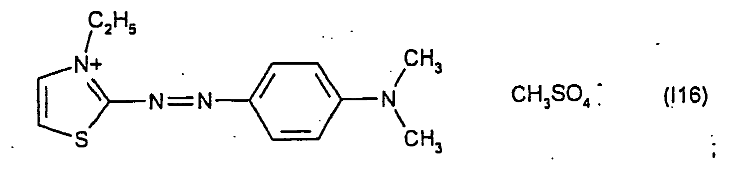 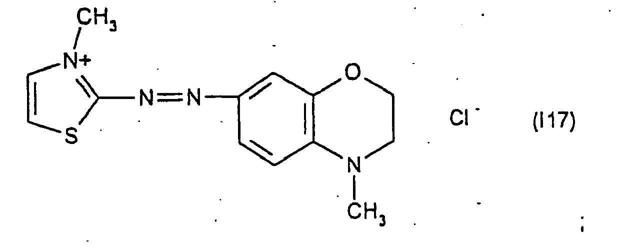 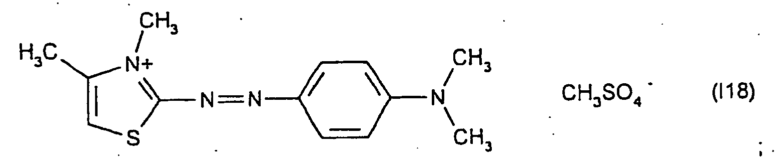 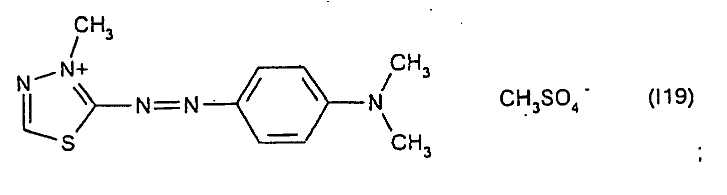 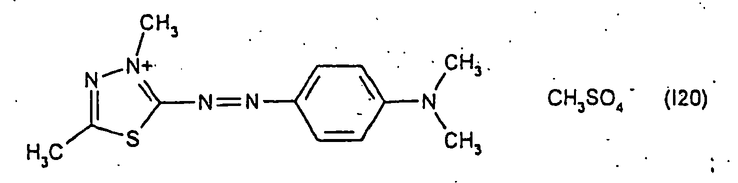 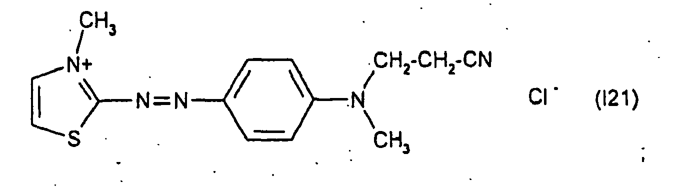 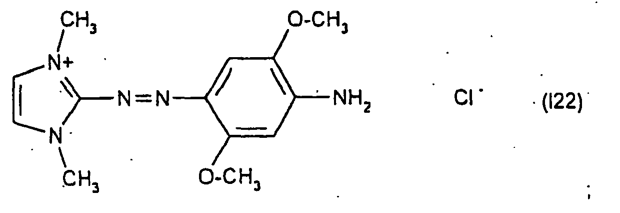 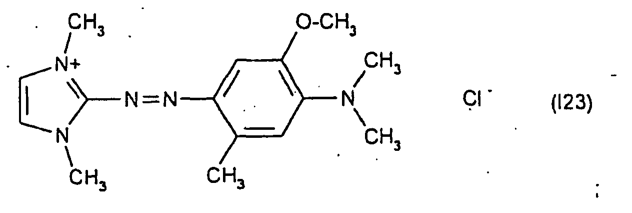 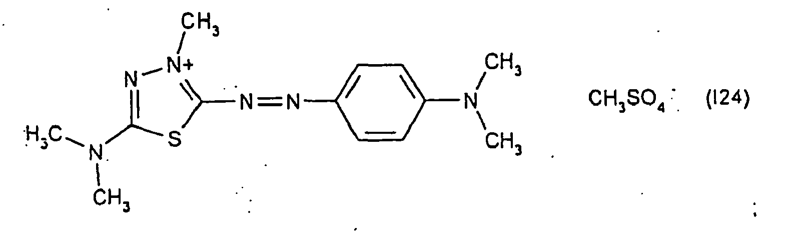 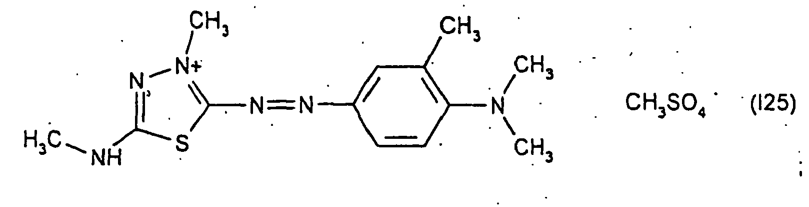 et

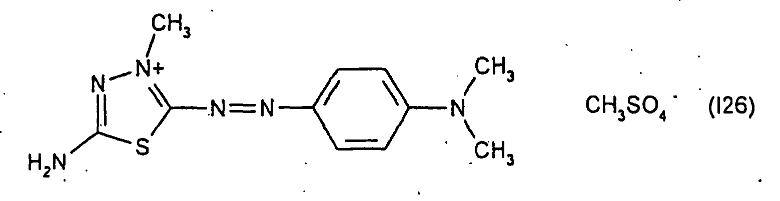

Parmi les composés particuliers de structures (I1) à (I26) décrits ci-dessus, on préfère tout particulièrement le composé répondant à la structure (I1).

Les sels d'addition avec un acide utilisables dans le cadre des compositions tinctoriales de invention sont notamment choisis parmi les chlorhydrates, les bromhydrates, les sulfates et les tartrates.

L'agent oxydant présent dans la composition tinctoriale est choisi parmi les agents oxydants classiquement utilisés en coloration d'oxydation et de préférence parmi le peroxyde d'hydrogène, le peroxyde d'urée, les bromates de métaux alcalins, les persels tels que les perborates et persulfates. Le peroxyde d'hydrogène est particulièrement préféré.

Le ou les colorants directs cationiques de formule (I) conformes à l'Invention, représentent de préférence de 0,001 à 10 % en poids environ du poids total de la composition tinctoriale prête à l'emploi et encore plus préférentiellement de 0,05 à 2 % en poids environ de ce poids.

La ou les bases d'oxydation conformes à l'invention, c'est à dire la ou les paraphénylènediamines de formule (II) et/ou la ou les bis-phénylaflcylènediamines de formule (III) représentent de préférence de 0,0001 à 10 % en poids environ du poids total de la composition tinctoriale prête à l'emploi et encore plus préférentiellement de 0,001 à 5 % en poids environ de ce poids.

Le ou les méta-phénylènediamines de formule (IV) conformes à l'invention représentent de préférence de 0,0001 à 5 % en poids environ du poids total de la composition tinctoriale prête à l'emploi et encore plus préférentiellement de 0,005 à 3 % en poids environ de ce poids.

Le pH de la composition tinctoriale telle que définie précédemment est généralement compris entre 5 et 12 environ. Il peut être ajusté à la valeur désirée au moyen d'agents acidifiants ou alcalinisants habituellement utilisés en teinture des fibres kératiniques.

Parmi les agents acidifiants, on peut citer, à titre d'exemples, les acides minéraux ou organiques comme l'acide chlorhydrique, l'acide orthophosphorique, les acides carboxyliques comme l'acide tartrique, l'acide citrique, l'acide lactique, les acides sulfoniques.

Parmi les agents alcalinisants, on peut citer, à titre d'exemples, l'ammoniaque, les carbonates alcalins, les alcanolamines telles que les mono-, di- et triéthanolamines ainsi que leurs dérivés, les hydroxydes de sodium ou de potassium et les composés de formule (VI) suivante : 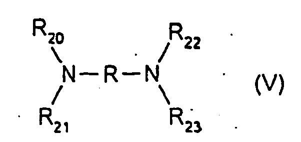 dans laquelle R est un reste propylène éventuellement substitué par un groupement hydroxyle ou un radical alkyle en C₁-C₄ ; R₂₀, R₂₁, R₂₂ et R₂₃, identiques ou différents, représentent un atome d'hydrogène, un radical alkyle en C₁-C₄ ou hydroxyalkyle en C₁-C₄.

La composition tinctoriale conforme à l'invention peut encore contenir, en plus des colorants définis ci-dessus, d'autres coupleurs et/ou des colorants directs, notamment pour modifier les nuances ou les enrichir en reflets.

Le milieu approprié pour la teinture (ou support) de la composition tinctoriale prête à l'emploi conforme à l'invention est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; la glycérol ; les glycols et éthers de glycols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, les produits analogues et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Les compositions tinctoriales prêtes à l'emploi conformes à l'invention peuvent également renfermer divers adjuvants utilisés classiquement dans les compositions pour la teinture des cheveux, tels que des agents tensio-actifs anioniques, cationiques, non-ioniques, amphotères, ou leurs mélanges, des polymères anioniques, cationiques, non-ioniques, amphotères, ou leurs mélanges, des agents épaississants minéraux ou organiques, des agents antioxydants, des agents de pénétration, des agents séquestrants, des parfums, des tampons, des agents dispersants, des agents de conditionnement, des agents filmogènes, des agents conservateurs, des agents opacifiants.

Bien entendu, l'homme de l'art veillera à choisir le ou les éventuels composés complémentaires mentionnés ci-avant de manière telle que les propriétés avantageuses attachées intrinsèquement à la composition tinctoriale prête à l'emploi conforme à l'invention ne soient pas, ou substantiellement pas, altérées par la ou les adjonctions envisagées.

Les compositions tinctoriales prêtes à l'emploi conformes à l'invention peuvent se présenter sous des formes diverses, telles que sous forme de liquides, de crèmes, de gels, ou sous toute autre forme appropriée pour réaliser une teinture des fibres kératiniques, et notamment des cheveux humains.

L'invention a également pour objet un procédé de teinture des fibres kératiniques et en particulier des fibres kératiniques humaines telles que les cheveux mettant en oeuvre la composition tinctoriale prête à l'emploi telle que définie précédemment.

Selon ce procédé, on applique sur les fibres la composition tinctoriale prête à l'emploi telle que définie précédemment, et on laisse poser pendant 3 à 40 minutes environ, de préférence 5 à 30 minutes environ, après quoi on rince, on lavé éventuellement au shampooing, on rince à nouveau et on sèche.

Selon une première forme de réalisation préférée, le procédé comporte une étape préliminaire consistant à stocker sous forme séparée, d'une part, une composition (A) comprenant, dans un milieu approprié pour la teinture, au moins une base d'oxydation choisie parmi les paraphénylènediamines, les bis-phénylalkylènediamines, et leurs sels d'addition avec un acide, au moins un coupleur choisi parmi les méta-phénylènediamines, et leurs sels d'addition avec un acide et au moins un colorant direct cationique choisi parmi les composés de formule (1) telle que définie précédemment et, d'autre part, une composition (B) renfermant, dans un milieu approprié pour la teinture, au moins un agent **oxy⁼** dant tel que défini précédemment, et à procéder à leur mélange au moment de l'emploi avant d'appliquer ce mélange sur les fibres kératiniques.

Selon une deuxième forme de réalisation préférée, le procédé comporte une étape préliminaire consistant à stocker sous forme séparée, d'une part, une composition (A) comprenant, dans un milieu approprié pour la teinture au moins une base d'oxydation choisie parmi les paraphénylènediamines, les bis-phénylalkylènediamines, et leurs sels d'addition avec un acide, au moins un coupleur choisi parmi les méta-phénylènediamines, et leurs sels d'addition avec un acide; d'autre part une composition (A') comprenant, dans un milieu approprié pour la teinture, au moins un colorant direct cationique choisi parmi les composés de formule (I) telle que définie précédemment ; et enfin, une composition (B) renfermant, dans un milieu approprié pour la teinture, au moins un agent oxydant tel que défini précédemment, et à procéder à leur mélange au moment de l'emploi avant d'appliquer ce mélange sur les fibres kératiniques.

La composition (A') utilisée selon cette deuxième variante du procédé conforme à l'invention, peut éventuellement se présenter sous forme de poudre, le ou les colorants directs cationiques de formule (1) conformes à l'invention constituant alors à lui (eux) seul(s) la totalité de ladite composition (A') ou étant éventuellement dispersé(s) dans un excipient pulvérulent organique et/ou minéral.

Lorsqu'il est présent dans la composition A', l'excipient organique peut être d'origine synthétique ou végétale et est choisi notamment parmi les polymères synthétiques réticulés et non réticulés, les polysaccharides comme les celluloses et les amidons modifiés ou non ainsi que les produits naturels les renfermant tels que la sciure de bois et les gommes végétales (guar, caroube, xanthane,).

Lorsqu'il est présent dans la composition (A'), l'excipient minéral peut être constitué par des oxydes métalliques tels que les oxydes de titane, les oxydes d'aluminium, le kaolin, le talc, les silicates, le mica et les silices.
Un excipient avantageusement préféré selon l'invention est la sciure de bois.

La composition (A') en poudre peut encore renfermer des liants ou des produits d'enrobage dans une quantité ne dépassant pas de préférence 3% en poids environ du poids total de ladite composition (A').

Ces liants sont de préférence choisis parmi les huiles et les corps gras liquides d'origine minérale, synthétique, animale ou végétale.

La composition (A') peut éventuellement encore contenir d'autres adjuvants, à l'état de poudre, en particulier des tensio-actifs de toute nature, des agents de conditionnement du cheveu comme par exemple des polymères cationiques.

Un autre objet de l'invention est un dispositif à plusieurs compartiments ou "kit" de teinture ou tout autre système de conditionnement à plusieurs compartiments dont un premier compartiment renferme la composition (A) telle que définie ci-dessus, un second compartiment éventuel renferme la composition (A') telle que définie ci-dessus lorsqu'elle est présente et un troisième compartiment renferme la composition oxydante (B) telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

Les exemples qui suivent sont destinés à illustrer l'invention.

### EXEMPLES

### EXEMPLE 1

On a préparé la composition 1 (A), conforme à l'invention, suivante (teneurs en grammes) :

| **COMPOSITION** | **1 (A)** |
|---|---|
| | |
| Paraphénylènediamine | 0,7 |
| | |
| Dichlorhydrate de 2,4-diamino 1-(β-hydroxyéthyloxy) benzène | 0,35 |
| | |
| Colorant cationique de structure (I1) | 0,4 |
| | |
| Support de teinture commun (*) | (*) |
| Eau q.s.p. | 100 g |

| | |
|---|---|
| (*) support de teinture commun: - Alcool oléique polyglycérolé à 2 moles de glycérol 4,0 g - Alcool oléique polyglycérolé à 4 moles de glycérol, à 78 % de matières actives (M.A.) 5,69 g M.A. - Acide oléique 3,0 g - Amine oléique à 2 moles d'oxyde d'éthylène vendue sous la dénomination commerciale ETHOMEEN 012.par la société AKZO 7,0 g - Laurylamino succinamate de diéthylaminopropyle, sel de sodium, à 55 % de M.A. 3,0 g M.A. - Alcool oléique 5,0 g - Diéthanolamide d'acide oléique 12,0 g - Propylèneglycol 3,5 g - Alcool éthylique 7,0 g - Dipropylèneglycol 0,5 g - Monométhyléther de propylèneglycol 9,0 g - Métabisulfite de sodium en solution aqueuse, à 35 % de M.A. 0,455 g M.A. - Acétate d'ammonium 0,8 g - Antioxydant, séquestrant q.s. - Parfum, conservateur q.s. - Ammoniaque à 20 % de NH₃ 10,0 g | |

Au moment de l'emploi, on a mélangé la composition 1 (A) avec une quantité égale d'une composition (B) constituée par une solution de peroxyde d'hydrogène à 20 volumes (6 % en poids).

La composition résultante (composition prête à l'emploi conforme à l'invention) a été appliquée pendant 30 minutes sur des mèches de cheveux gris naturels à 90 % de blancs. Les mèches de cheveux ont ensuite été rincées, lavées avec un shampooing standard puis séchées.

Les cheveux ont été teints dans une nuance châtain cendré lumineuse résistant bien aux shampooings ultérieurs.

Selon une variante de l'invention, la colorant direct cationique de structure (I1) peut être incorporé dans la composition 1 (A) au moment de l'emploi.

### EXEMPLE 2

On a préparé la composition 2 (A) suivante :
- Sulfate de paratoluylènediamine 1,25 g
- Dichlorhydrate de 2-amino 4-N-(β-hydroxyéthyl)amino 1-méthoxy benzène 0,35 g

- Support de teinture commun tel que décrit précédemment pour l'exemple 1 (*)
- Eau déminéralisée q.s.p. 100 g

On a préparé la composition 2 (A') suivante :

| | |
|---|---|
| - Colorant cationique de structure (I1) | 4 g |
| - Polyammonium quaternaire vendu sous la dénomination commerciale CELQUAT SC-240 par la société National Starch | 10 g |
| - Sdure de bois q.s.p. | 100 g |

Au moment de l'emploi, on a mélangé une partie en poids de la composition 2 (A) ci-dessus avec 0,1 partie en poids de la composition 2 (A') et avec une partie en poids d'une composition (B) constituée par une solution de peroxyde d'hydrogène à 20 volumes (6 % en poids).

La composition résultante a été appliquée pendant 30 minutes sur des mèches de cheveux gris naturels à 90 % de blancs. Les cheveux ont ensuite été rincées, lavées avec un shampooing standard puis séchées.

Les cheveux ont été teints dans une nuance châtain cendré résistant bien aux shampooings ultérieurs.

## Revendications

1. Composition prête à l'emploi pour la teinture d'oxydation des fibres kératiniques et en particulier des fibres kératiniques humaines telles que les cheveux **caractérisée par le fait qu'**elle comprend, dans un milieu approprié pour la teinture
- au moins une base d'oxydation choisie parmi les paraphénylènediamines, les bis-phénylalkylènediamines, et leur sels d'addition avec un acide,
- au moins un coupleur choisi parmi les méta-phénylènediamines, et leurs sels d'addition avec un acide,
- au moins un colorant direct cationique choisi parmi les composés de formule (I) suivante: 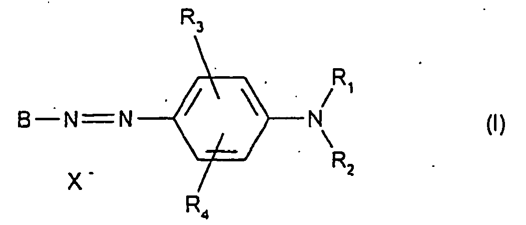
dans laquelle:
R₁ représente un atome d'hydrogène ou un radical alkyle en C₁-C₄,
R₂ représente un atome d'hydrogène, un radical alkyle pouvant être substitué par un radical -CN ou par un groupement amino, un radical 4'-aminophényle ou forme avec R₁ ou avec un atome de carbone du noyau benzénique supportant les radicaux R₃ et R₄. un hétérocycle éventuellement oxygéné et/ou azoté pouvant être substitué par un radical alkyle en C₁-C₄.
R₃ et R₄, identiques ou différents, représentent un atome d'hydrogène, un atome d'halogène tel que le brome, le chlore, l'iode ou le fluor, un radical alkyle en C₁-C₄ ou alcoxy en C₁-C₄, un radical -CN,
X⁻ représente un anion de préférence choisi parmi le chlorure, le méthyl sulfate et l'acétate,
B représente un groupement choisi par les structures B1 à B11 suivantes : 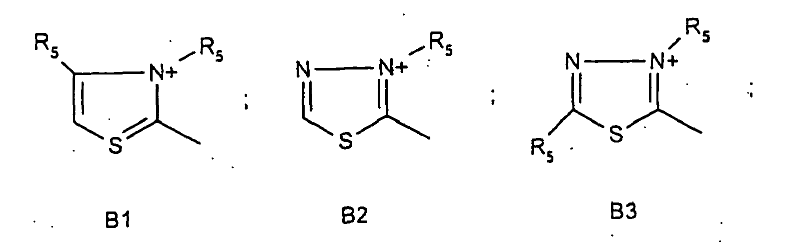 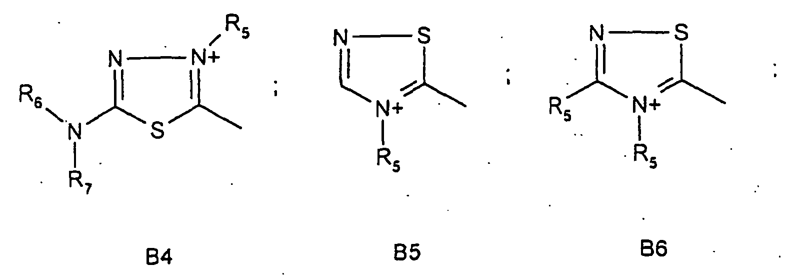 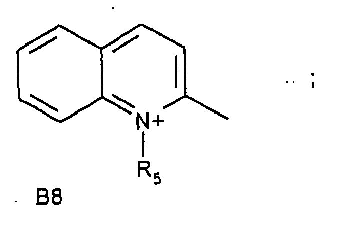 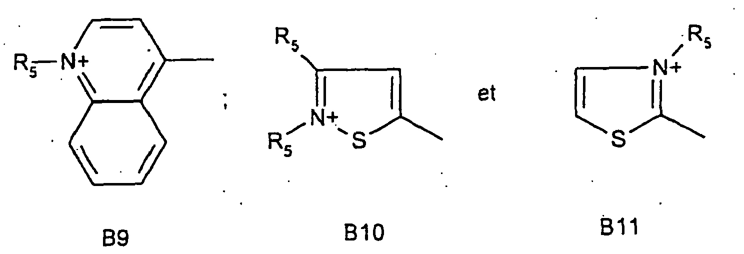
dans lesquelles R₅ représente un radical alkyle en C₁-C₄, R₆ et R₇, identiques ou différents, représentent un atome d'hydrogène ou un radical alkyle en C₁-C₄ ;
lorsque R₁ et R₂ forment un hétérocycle azoté, ou lorsque R₃ et R₄ représentent un radical alkyle en C₁-C₄ ou un radical alcoxy en C₁-C₄, ou lorsque R₂ représente un radical 4'-aminaphényle, alors B peut également représenter un groupement de structure B12 suivante: 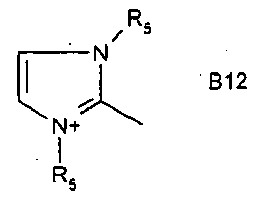 dans laquelle R₅ a la même signification que celle indiquée ci-dessus pour les structures B1 à B11 ; et
- au moins un agent oxydant.

2. Composition selon la revendication 1, **caractérisée par le fait que** les paraphénylènediamines sont choisies parmi les composés de formule (II) suivante, et leur sels d'addition avec un acide : 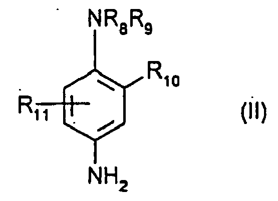 dans laquelle :
R₈ représente un atome d'hydrogène, un radical alkyle en C₁-C₄, monohydroxyalkyle en C₁-C₄, polyhydroxyalkyle en C₂-C₄, phényle, 4'-aminophényle ou alcoxy(C₁-C₄)alkyle en C₁-C₄,
R₉ représente un atome d'hydrogène, un radical alkyle en C₁-C₄, monohydroxyalkyle en C₁-C₄ ou polyhydroxyalkyle en C₂-C₄.
R₁₀ représente un atome d'hydrogène, un atome d'halogène tel qu'un atome de chlore, de brome, diode ou de fluor, un radical alkyle en C₁-C₄, monohydroxyalkyle en C₁-C₄, hydroxyalcoxy en C₁-C₄, mésylaminoalcoxy en C₁-C₄, carbamoylaminoalcoxy en C₁-C₄ ou acétylaminoalcoxy en C₁-C₄,
R₁₁ représente un atome d'hydrogène ou un radical alkyle en C₁-C₄.

3. Composition selon la revendication 2, **caractérisée par le fait que** les paraphénylènediamines de formule (11) sont choisies parmi la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-amino N,N-bis-(β-hydroxyéthyl) 3-méthyl aniline, la 4-amino 3-chloro N,N-bis-(β-hydroxyéthyl) aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, et leurs sels d'addition avec un acide.

4. Composition selon la revendication 1, **caractérisée par le fait que** les bis-phénylalkylénediamines sont choisies parmi les composés de formule (III) suivante, et leurs sels d'addition avec un acide: 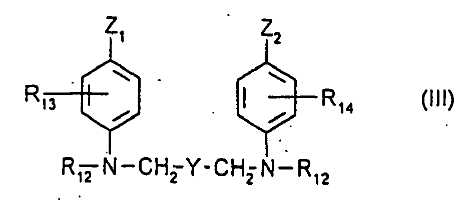 dans laquelle:
Z₁ et Z₂, identiques ou différents, représentent un radical hydroxyle ou NHR₁₅ dans lequel R₁₅ représente un atome d'hydrogène ou un radical alkyle en C₁-C₄.
R₁₂ représenté un atome d'hydrogène, un radical alkyle en C₁-C₄, monohydroxyalkyle en C₁-C₄, polyhydroxyalkyle en C₂-C₄ ou aminoalkyle en C₁-C₄ dont le reste amino peut être substitué,
R₁₃ et R₁₄, identiques ou différents, représentent un atome d'hydrogène ou d'halogène ou un radical alkyle en C₁-C₄.
Y représente un radical pris dans le groupe constitué par les radicaux suivants : -(CH₂)ₙ ; -(CH₂)ₘ-O-(CH₂)ₘ; (CH₂)ₘ-CHOH-(CH₂)ₘ et 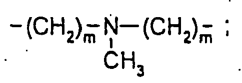
dans lesquels n est un nombre entier compris entre 0 et 8 inclusivement et m est un nombre entier compris entre 0 et 4 inclusivement

5. Composition selon la revendication 4, **caractérisée par le fait que** les bis-phénylalkylènediamines de formule (III) sont choisies parmi le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N'N'-bis-(β-hydroxyéthyl) N,N'-bis-(4-aminophényl) tétraméthylènediamine, la N,N'-bis-(4-méthylaminophényl) tétraméthylènediamine, la N,N'-bis-(éthyl) N,N'-bis-(4'-amino, 3'-méthylphényl) éthylènediamine, et leurs sels d'addition avec un acide.

6. Composition selon rune quelconque des revendications précédentes, **caractérisée par le fait que** les méta-phénylènediamines sont choisies parmi les composés de formule (IV) suivante, et leurs sels d'addition avec un acide: 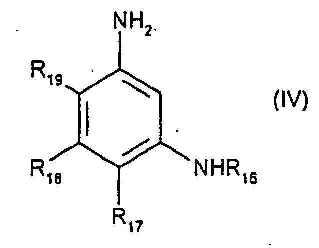 dans laquelle :
- R₁₆ représente un atome d'hydrogène, un radical alkyle en C₁-C₄, monohydroxyalkyle en C₁-C₄ ou polyhydroxyalkyle en C₂-C₄ :
- R₁₇ et R₁₈, identiques ou différents, représentent un atome d'hydrogène, un radical alkyle en C₁-C₄, monohydroxyalcoxy en C₁-C₄ ou polyhydroxyalcoxy en C₂-C₄ ;
- R₁₉ représente un atome d'hydrogène, un radical alkoxy en C₁-C₄, aminoalkoxy en C₁-C₄, monohydroxyalkoxy en C₁-C₄, polyhydroxyalkoxy en C₂-C₄ ou un radical 2,4-diaminophénoxyalkoxy.

7. Composition selon la revendication 6, **caractérisée par le fait que** les méta-phénylènediamines de formule (IV) sont choisies parmi la métaphénylènediamine, le 3,5-diamino 1-éthyl 2-méthoxybenzène, le 3,5-diamino 2-méthoxy 1-méthyl benzène, le 2,4-diamino 1-éthoxybenzène, le 1,3-bis-(2,4-diaminophénoxy) propane, le bis-(2,4-diaminophénoxy) méthane, le 1-(β-aminoéthyloxy) 2,4-diamino benzène, le 2-amino 1-(β-hydroxyéthyloxy) 4-méthylamino benzène, le 2,4-diamino 1-éthoxy 5-méthyl benzène, le 2,4-diamino 5-(β-hydroxyéthyloxy) 1-méthylbenzène, le 2,4-diamino 1-(β,γ-dihydroxypropyloxy) benzène, le 2,4-damino 1-(β-hydroxyéthyloxy) benzène, le 2-amino 4-N-(β-hydroxyéthyl) amino 1-méthoxy benzène, et leurs sels d'addition avec un acide.

8. Composition selon l'une quelconque des revendications précédentes, **caractérisée par le fait que** les colorants directs cationiques de formule (I) sont choisis parmi les composés répondant aux structures (I1) à (I26) suivantes : 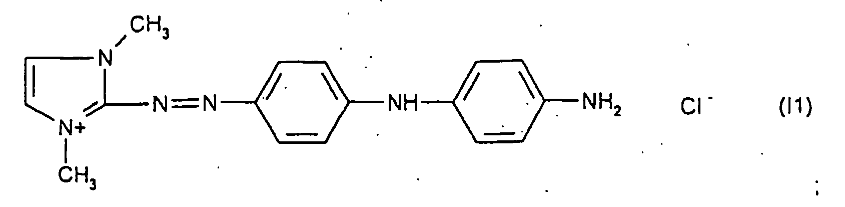 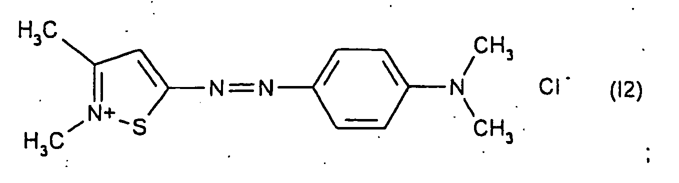 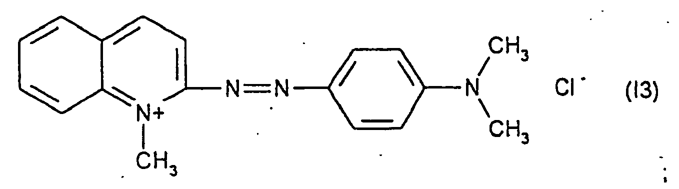 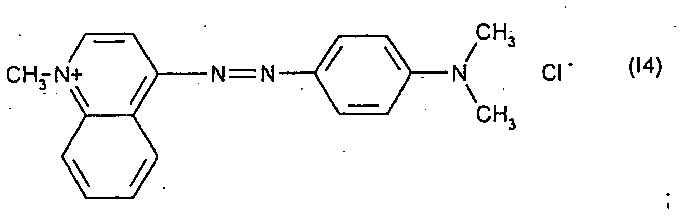 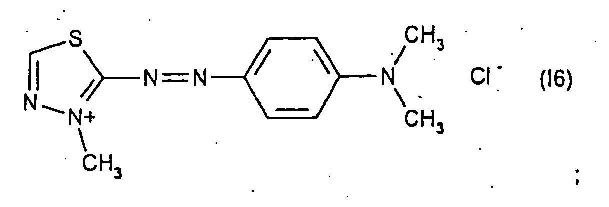 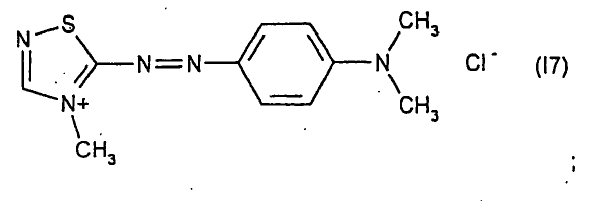 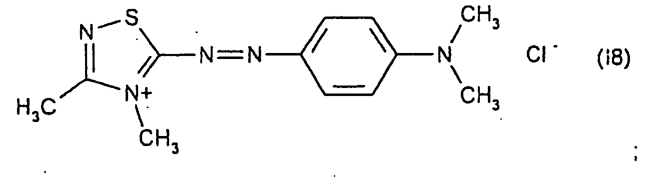 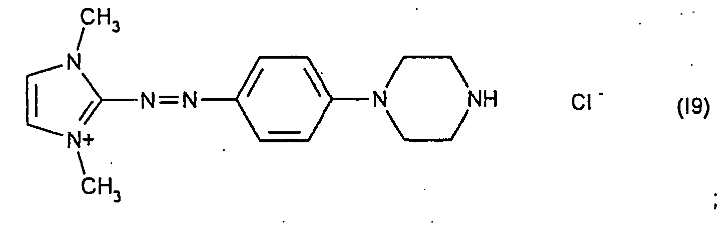 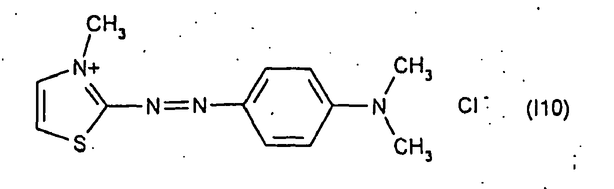 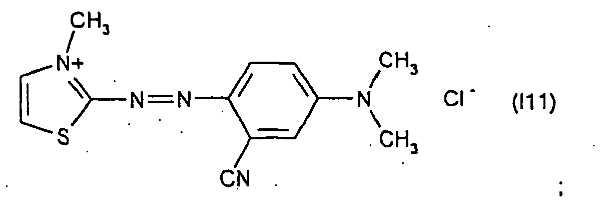 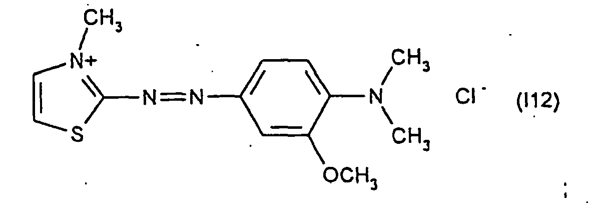 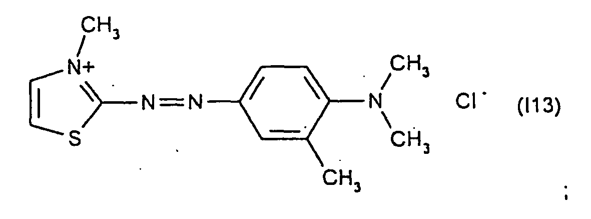 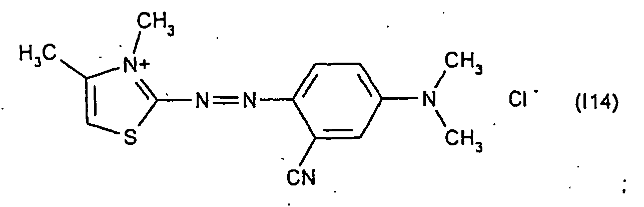 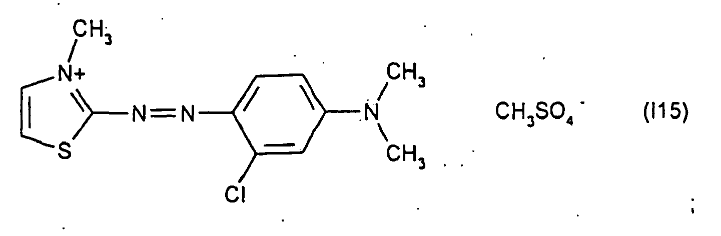 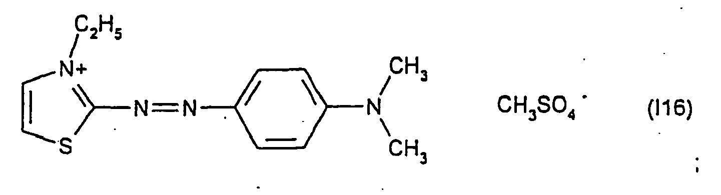 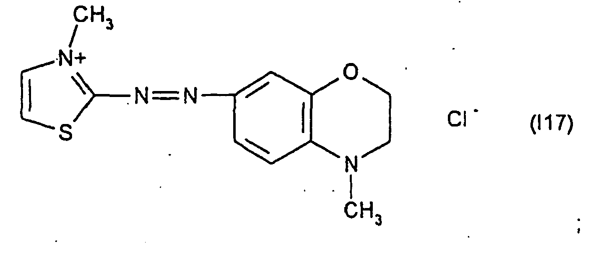 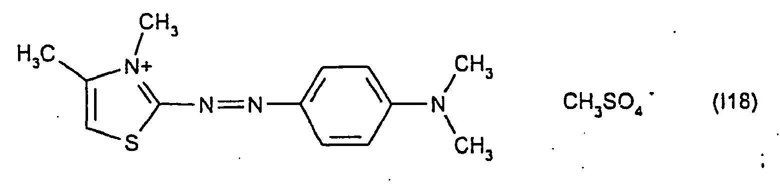 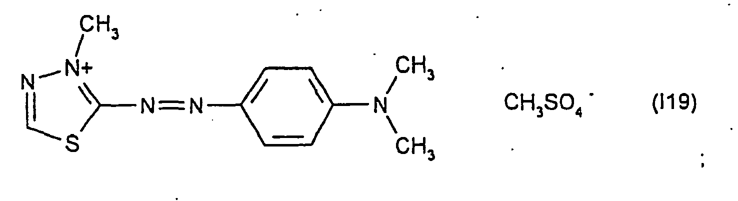 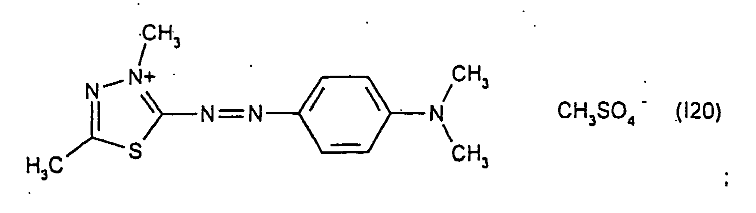 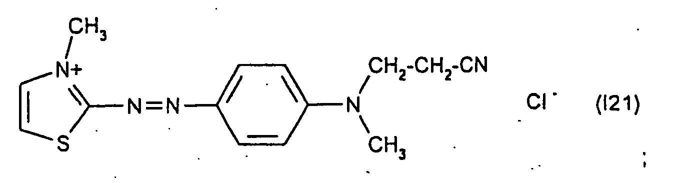 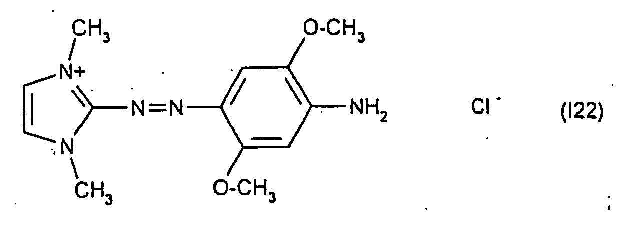 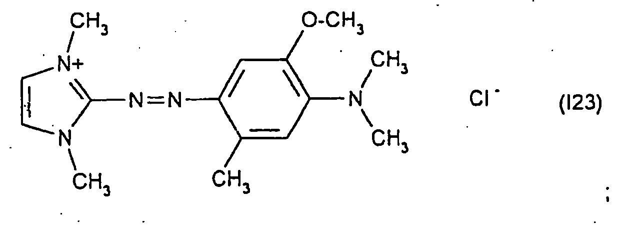 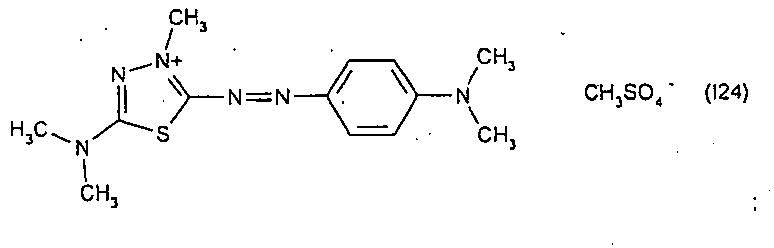 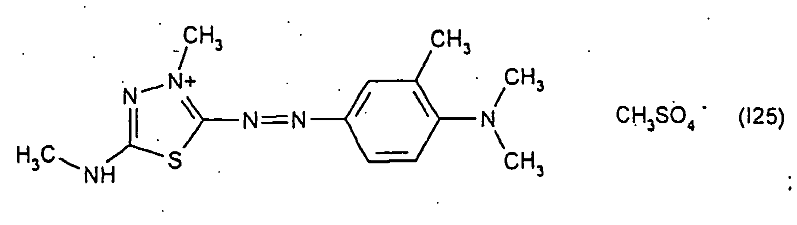 et

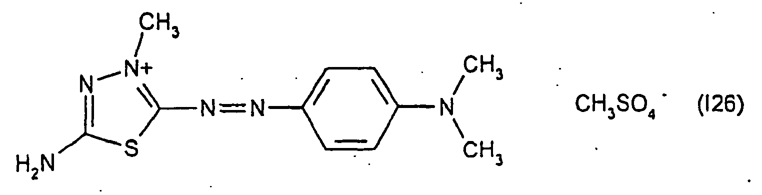

9. Composition selon l'une quelconque des revendications précédentes, **caractérisée par le fait que** les sels d'addition avec un acide sont choisis parmi les chlorhydrates, les bromhydrates, les sulfates et les tartrates.

10. Composition selon l'une quelconque des revendications précédentes, **caractérisée par le fait que** l'agent oxydant est choisi parmi le peroxyde d'hydrogène, le peroxyde d'urée, les bromates de métaux alcalins, les persels tels que les perborates et persulfates

11. Composition selon l'une quelconque des revendications précédentes, **caractérisée par le fait que** le ou les colorants directs cationiques de formule (I) représentent de 0,001 à 10 % en poids du poids total de la composition tinctoriale prête à l'emploi.

12. Composition selon l'une quelconque des revendications précédentes, **caractérisée par le fait que** la ou les paraphénylènediamines de formule (II) et/ou la ou les bis-phénylalkylènediamines de formule (III) représentent de 0,0001 à 10 % en poids du poids total de la composition tinctoriale prête à l'emploi.

13. Composition selon l'une quelconque des revendications précédentes, **caractérisée par le fait que** le ou les méta-phénylènediamines de formule (IV) représentent de 0,0001 à 5 % en poids du poids total de la composition tinctoriale prête à l'emploi.

14. Composition selon l'une quelconque des revendications précédentes, **caractérisée par le fait qu'**elle présente un pH compris entre 5 et 12.

15. Composition selon l'une quelconque des revendications précédentes, **caractérisée par le fait que** le milieu approprié pour la teinture est constitué par de l'eau ou par un mélange d'eau et d'au moins un solvant organique.

16. Procédé de teinture des fibres kératiniques et en particulier des fibres kératiniques humaines telles que les cheveux **caractérisé par le fait que** l'on applique sur ces fibres au moins une composition tinctoriale telle que définie dans l'une quelconque des revendications 1 à 15.

17. Procédé selon la revendication 16, **caractérisé par le fait qu'**il comporte une étape préliminaire consistant à stocker sous forme séparée, d'une part, une composition (A) comprenant, dans un milieu approprié pour la teinture, au moins une base d'oxydation choisie parmi les paraphénylènediamines, les bis-phénylalkylènediamines, et leurs sels d'addition avec un acide, au moins un coupleur choisi parmi les méta-phénylènediamines, et leurs sels d'addition avec un acide, et au moins un colorant direct cationique choisi parmi les composés de formule (I) tels que définis dans la revendication 1 et d'autre part, une composition (B) renfermant, dans un milieu approprié pour la teinture, au moins un agent oxydant, et à procéder à leur mélange au moment de l'emploi avant d'appliquer ce mélange sur les fibres kératiniques.

18. Procédé de teinture selon la revendication 16, **caractérisé par le fait qu'**il comporte une étape préliminaire consistant à stocker sous forme séparée, d'une part, une composition (A) comprenant, dans un milieu approprié pour la teinture au moins une base d'oxydation choisie parmi les paraphénylènediamines, les bis-phénylalkylènediamines, et leurs sels d'addition avec un acide, au moins un coupleur choisi parmi les méta-phénylènediamines, et leurs sels d'addition avec un acide ; d'autre part une composition (A') comprenant, dans un milieu approprié pour la teinture, au moins un colorant direct cationique choisi parmi les composés de formule (I) tels que définis dans la revendication 1 ; et enfin, une composition (B) renfermant, dans un milieu approprié pour la teinture, au moins un agent oxydant et à procéder à leur mélange au moment de l'emploi avant d'appliquer ce mélange sur les fibres kératiniques.

19. Procédé selon la revendication 18, **caractérisé par le fait que** la composition (A') se présente sous forme de poudre.

20. Dispositif à plusieurs compartiments ou "kit" de teinture, **caractérisé par le fait qu'**un premier compartiment renferme la composition (A) telle que définie à la revendication 17 et un second compartiment renferme une composition oxydante (B).

21. Dispositif à plusieurs compartiments ou "kit" de teinture, **caractérisé par le fait qu'**un premier compartiment renferme une composition (A) telle que définie à la revendication 18, un second compartiment referme une composition (A') telle que définie à la revendication 18 ou 19 et un troisième compartiment renferme une composition oxydante (B).

## Claims

1. Ready-to-use composition for the oxidation dyeing of keratin fibres, and in particular human keratin fibres such as the hair, **characterized in that** it comprises, in a medium which is suitable for dyeing:
- at least one oxidation base chosen from para-phenylenediamines and bis(phenyl)alkylenediamines, and the addition salts thereof with an acid,
- at least one coupler chosen from meta-phenylenediamines, and the addition salts thereof with an acid,
- at least one cationic direct dye chosen from the compounds of formula (I) below: 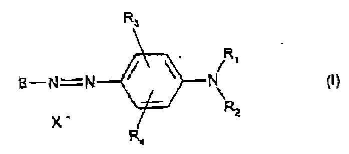
in which:
R₁ represents a hydrogen atom, or a C₁-C₄ alkyl radical,
R₂ represents a hydrogen atom, an alkyl radical which may be substituted with a -CN radical or with an amino group, a 4'-aminophenyl radical or forms, with R₁ or with a carbon atom of the benzene ring supporting the radicals R₃ and R₄, an optionally oxygenated and/or nitrogenous heterocycle which may be substituted with a C₁-C₄ alkyl radical,
R₃ and R₄, which may be identical or different, represent a hydrogen atom, a halogen atom such as bromine, chlorine, iodine or fluorine, a C₁-C₄ alkyl or C₁-C₄ alkoxy radical or a -CN radical,
X⁻ represents an anion preferably chosen from chloride, methylsulphate and acetate,
B represents a group chosen from the structures B1 to B11 below: 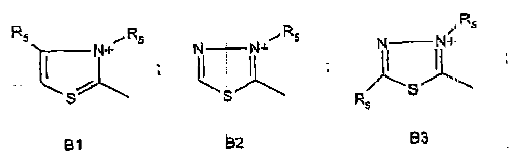 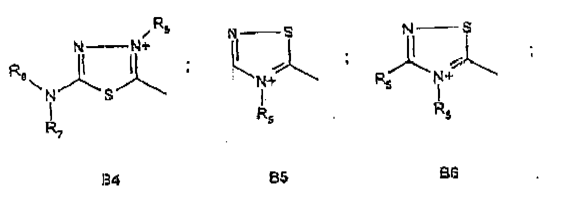 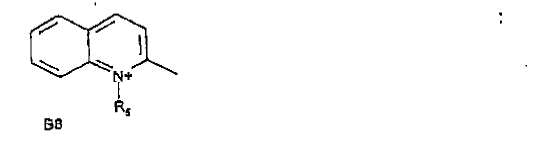 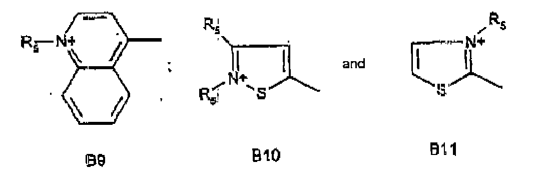
in which R₅ represents a C₁-C₄ alkyl radical, R₆ and R₇, which may be identical or different, represent a hydrogen atom or a C₁-C₄ alkyl radical;
when R₁ and R₂ form a nitrogenous heterocycle, or when R₃ and R₄ represent a C₁-C₄ alkyl radical or a C₁-C₄ alkoxy radical, or when R₂ represents a 4'-aminophenyl radical, then B can also represent a group of structure B12 below: 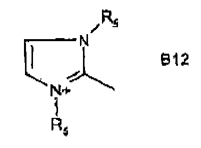 in which R₅ has the same meaning as that indicated above for the structures B1 to B11; and
- at least one oxidizing agent.

2. Composition according to Claim 1, **characterized in that** the para-phenylenediamines are chosen from the compounds of formula (II) below and the addition salts thereof with an acid: 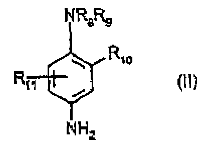 in which:
R₈ represents a hydrogen atom, a C₁-C₄ alkyl, C₁-C₄ monohydroxyalkyl, C₂-C₄ polyhydroxyalkyl, phenyl, 4'-aminophenyl or (C₁-C₄)alkoxy(C₁-C₄)alkyl radical,
R₉ represents a hydrogen atom or a C₁-C₄ alkyl, C₁-C₄ monohydroxyalkyl or C₂-C₄ polyhydroxyalkyl radical,
R₁₀ represents a hydrogen atom, a halogen atom such as a chlorine, bromine, iodine or fluorine atom, a C₁-C₄ alkyl, C₁-C₄ monohydroxyalkyl, C₁-C₄ hydroxyalkoxy, C₁-C₄ mesylaminoalkoxy, C₁-C₄ carbamoylaminoalkoxy or C₁-C₄ acetylaminoalkoxy radical,
R₁₁ represents a hydrogen atom or a C₁-C₄ alkyl radical.

3. Composition according to Claim 2, **characterized in that** the para-phenylenediamines of formula (II) are chosen from para-phenylenediamine, para-toluylenediamine, 2-chloro-para-phenylenediamine, 2,3-dimethyl-para-phenylenediamine, 2,6-dimethyl-para-phenylenediamine, 2,6-diethyl-para-phenylenediamine, 2,5-dimethyl-para-phenylenediamine, N,N-dimethyl-para-phenylenediamine, N,N-diethyl-para-phenylenediamine, N,N-dipropyl-para-phenylenediamine, 4-amino-N,N-diethyl-3-methylaniline, N,N-bis(β-hydroxyethyl)-para-phenylenediamine, 4-amino-N,N-bis(β-hydroxyethyl)-3-methylaniline, 4-amino-3-chloro-N,N-bis(β-hydroxyethyl)aniline, 2-β-hydroxyethyl-para-phenylenediamine, 2-fluoro-para-phenylenediamine, 2-isopropyl-para-phenylenediamine, N-(β-hydroxypropyl)-para-phenylenediamine, 2-hydroxymethyl-para-phenylenediamine, N,N-dimethyl-3-methyl-para-phenylenediamine, N,N-(ethyl-β-hydroxyethyl)-para-phenylenediamine, N-(β,γ-dihydroxypropyl)-para-phenylenediamine, N-(4'-aminophenyl)-para-phenylenediamine, N-phenyl-para-phenylenediamine, 2-β-hydroxyethyloxy-para-phenylenediamine and 2-β-acetylaminoethyloxy-para-phenylenediamine, and the addition salts thereof with an acid.

4. Composition according to Claim 1, **characterized in that** the bis(phenyl)alkylenediamines are chosen from the compounds of formula (III) below, and the addition salts thereof with an acid: 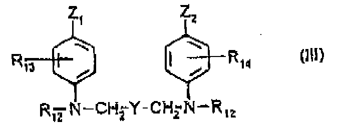 in which:
Z₁ and Z₂, which may be identical or different, represent a hydroxyl radical or NHR₁₅ in which R₁₅ represents a hydrogen atom or a C₁-C₄ alkyl radical,
R₁₂ represents a hydrogen atom or a C₁-C₄ alkyl, C₁-C₄ monohydroxyalkyl or C₂-C₄ polyhydroxyalkyl radical or a C₁-C₄ aminoalkyl radical in which the amino residue can be substituted,
R₁₃ and R₁₄, which may be identical or different, represent a hydrogen or halogen atom or a C₁-C₄ alkyl radical,
Y represents a radical taken from the group consisting of the following radicals: -(CH₂)ₙ; -(CH₂)ₘ-O-(CH₂)ₘ; - (CH₂)ₘ-CHOH- (CH₂)ₘ and 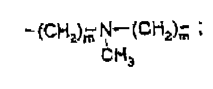
in which n is an integer between 0 and 8 inclusive and m is an integer between 0 and 4 inclusive.

5. Composition according to Claim 4, **characterized in that** the bis(phenyl)alkylenediamines of formula (III) are chosen from N,N'-bis(β-hydroxyethyl)-N,N'-bis(4'-aminophenyl)-1,3-diaminopropanol, N,N'-bis(β-hydroxyethyl)-N,N'-bis(4'-aminophenyl)ethylenediamine, N,N'-bis(4-aminophenyl)-tetramethylenediamine, N,N'-bis(β-hydroxyethyl)-N,N'-bis(4-aminophenyl)-tetramethylenediamine, N,N'-bis(4-methylaminophenyl)-tetramethylenediamine and N,N'-bis(ethyl)-N,N'-bis(4'-amino-3'-methylphenyl)ethylenediamine, and the addition salts thereof with an acid.

6. Composition according to any one of the preceding claims, **characterized in that** the meta-phenylenediamines are chosen from the compounds of formula (IV) below, and the addition salts thereof with an acid: 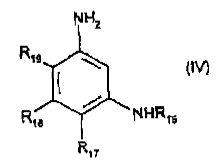 in which:
- R₁₆ represents a hydrogen atom or a C₁-C₄ alkyl, C₁-C₄ monohydroxyalkyl or C₂-C₄ polyhydroxyalkyl radical;
- R₁₇ and R₁₈, which may be identical or different, represent a hydrogen atom or a C₁-C₄ alkyl, C₁-C₄ monohydroxyalkoxy or C₂-C₄ polyhydroxyalkoxy radical;
- R₁₉ represents a hydrogen atom, a C₁-C₄ alkoxy, C₁-C₄ aminoalkoxy, C₁-C₄ monohydroxyalkoxy or C₂-C₄ polyhydroxyalkoxy radical or a 2,4-diaminophenoxyalkoxy radical.

7. Composition according to Claim 6, **characterized in that** the meta-phenylenediamines of formula (IV) are chosen from meta-phenylenediamine, 3,5-diamino-1-ethyl-2-methoxybenzene, 3,5-diamino-2-methoxy-1-methylbenzene, 2,4-diamino-1-ethoxybenzene, 1,3-bis(2,4-diaminophenoxy)propane, bis(2,4-diaminophenoxy)methane, 1-(β-aminoethyloxy)-2,4-diaminobenzene, 2-amino-1-(β-hydroxyethyloxy)-4-methylaminobenzene, 2,4-diamino-1-ethoxy-5-methylbenzene, 2,4-diamino-5-(β-hydroxyethyloxy)-1-methylbenzene, 2,4-diamino-1-(β,γ-dihydroxypropyloxy)benzene, 2,4-diamino-1-(β-hydroxyethyloxy)benzene and 2-amino-4-N-(β-hydroxyethyl)amino-1-methoxybenzene, and the addition salts thereof with an acid.

8. Composition according to any one of the preceding claims, **characterized in that** the cationic direct dyes of formula (I) are chosen from the compounds corresponding to structures (I1) to (I26) below: 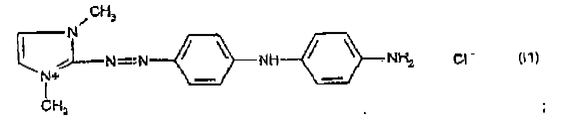 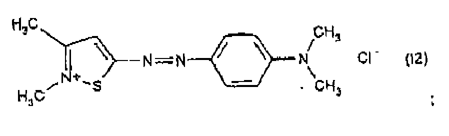 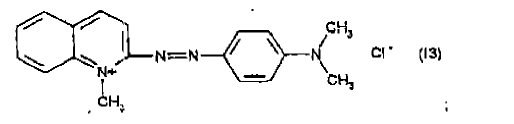 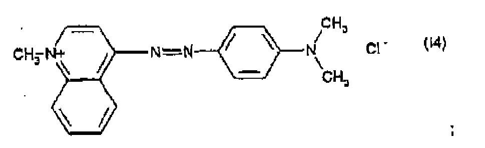 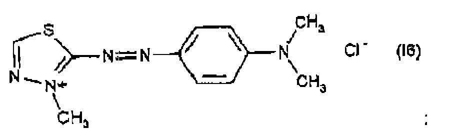 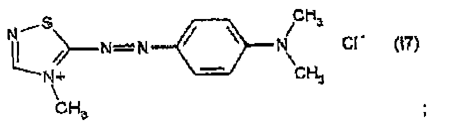 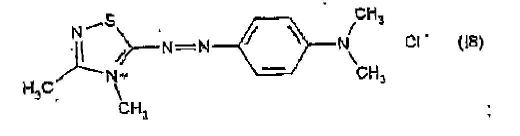 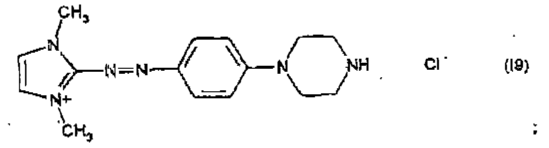 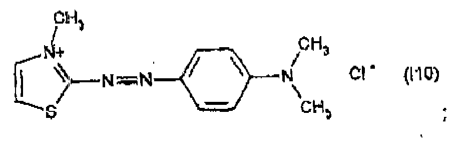 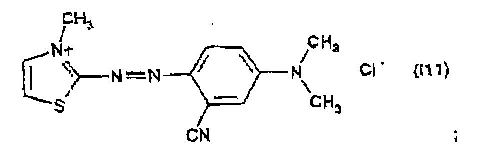 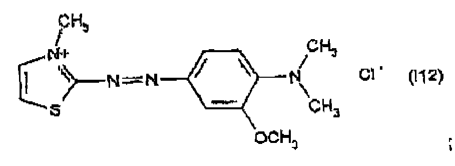 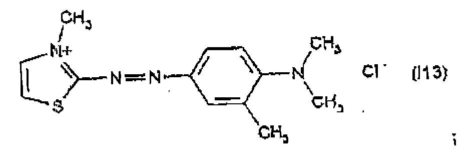 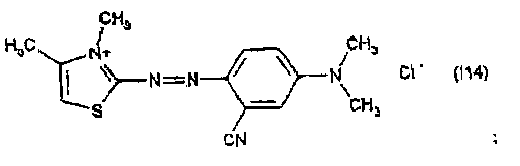 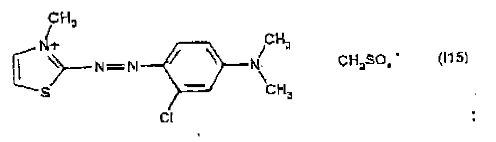 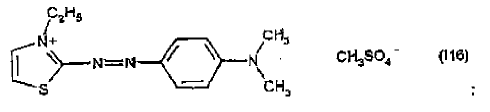 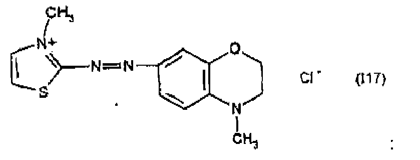 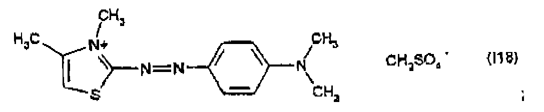 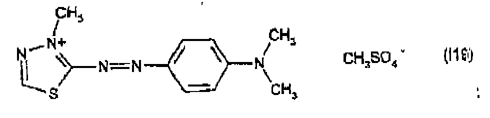 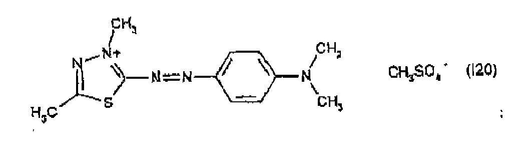 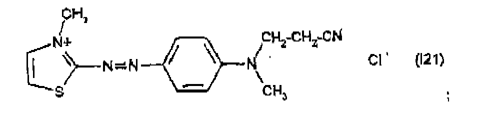 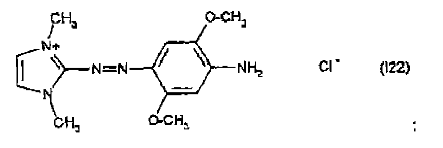 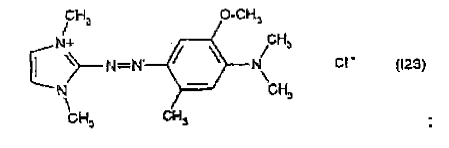 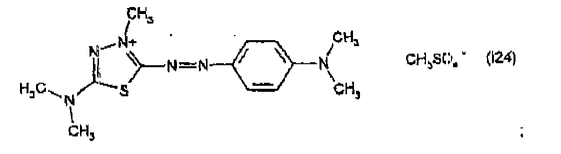 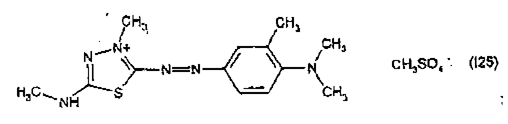 and

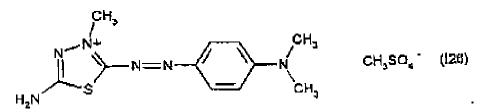

9. Composition according to any one of the preceding claims, **characterized in that** the addition salts with an acid are chosen from the hydrochlorides, hydrobromides, sulphates and tartrates.

10. Composition according to any one of the preceding claims, **characterized in that** the oxidizing agent is chosen from hydrogen peroxide, urea peroxide, alkali metal bromates and persalts such as perborates and persulphates.

11. Composition according to any one of the preceding claims, **characterized in that** the cationic direct dye(s) of formula (I) represent(s) from 0.001 to 10% by weight relative to the total weight of the ready-to-use dye composition.

12. Composition according to any one of the preceding claims, **characterized in that** the para-phenylenediamine(s) of formula (II) and/or the bis(phenyl)-alkylenediamine(s) of formula (III) represent(s) from 0.0001 to 10% by weight relative to the total weight of the ready-to-use dye composition.

13. Composition according to any one of the preceding claims, **characterized in that** the meta-phenylenediamine(s) of formula (IV) represent(s) from 0.0001 to 5% by weight relative to the total weight of the ready-to-use dye composition.

14. Composition according to any one of the preceding claims, **characterized in that** it has a pH of between 5 and 12.

15. Composition according to any one of the preceding claims, **characterized in that** the medium which is suitable for dyeing consists of water or of a mixture of water and at least one organic solvent.

16. Process for dyeing keratin fibres, and in particular human keratin fibres such as the hair, **characterized in that** at least one dye composition as defined in any one of Claims 1 to 15 is applied to these fibres.

17. Process according to Claim 16, **characterized in that** it includes a preliminary step which consists in separately storing, on the one hand, a composition (A) comprising, in a medium which is suitable for dyeing, at least one oxidation base chosen from para-phenylenediamines and bis(phenyl)alkylenediamines, and the addition salts thereof with an acid, at least one coupler chosen from meta-phenylenediamines, and the addition salts thereof with an acid, and at least one cationic direct dye chosen from the compounds of formula (I), as defined in Claim 1, and, on the other hand, a composition (B) containing, in a medium which is suitable for dyeing, at least one oxidizing agent, and in mixing them together at the time of use before applying this mixture to the keratin fibres.

18. Dyeing process according to Claim 16, **characterized in that** it includes a preliminary step which consists in separately storing, on the one hand, a composition (A) comprising, in a medium which is suitable for dyeing, at least one oxidation base chosen from para-phenylenediamines and bis(phenyl)alkylenediamines, and the addition salts thereof with an acid, at least one coupler chosen from meta-phenylenediamines, and the addition salts thereof with an acid; on the other hand, a composition (A') comprising, in a medium which is suitable for dyeing, at least one cationic direct dye chosen from the compounds of formula (I), as defined in Claim 1; and, lastly, a composition (B) containing, in a medium which is suitable for dyeing, at least one oxidizing agent, and in mixing them together at the time of use before applying this mixture to the keratin fibres.

19. Process according to Claim 18, **characterized in that** the composition (A') is in powder form.

20. Multi-compartment dyeing device or "kit", **characterized in that** a first compartment contains the composition (A) as defined in Claim 17 and a second compartment contains an oxidizing composition (B).

21. Multi-compartment dyeing device or "kit", **characterized in that** a first compartment contains a composition (A) as defined in Claim 18, a second compartment contains a composition (A') as defined in Claim 18 or 19, and a third compartment contains an oxidizing composition (B).

## Patentansprüche

1. Gebrauchsfertige Zusammensetzung für die oxidative Färbung von Keratinfasern und insbesondere menschlicher Keratinfasern wie den Haaren, die **dadurch gekennzeichnet ist, daß** sie in einem für die Färbung geeignetem Medium enthält:
- mindestens eine Oxidationsbase, die unter p-Phenylendiaminen, Bisphenylalkylendiaminen und deren Additionssalzen mit einer Säure ausgewählt ist,
- mindestens einen Kuppler, der unter m-Phenylendiaminen und deren Additionssalzen mit einer Säure ausgewählt ist,
- mindestens einen direktziehenden kationischen Farbstoff der folgenden Formel (I) 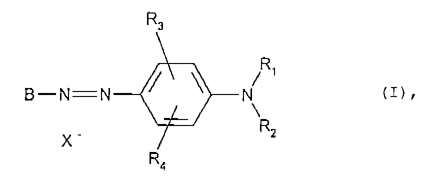 in der bedeuten:
- R₁ Wasserstoff oder eine C₁₋₄-Alkylgruppe,
- R₂ Wasserstoff, eine Alkylgruppe, die gegebenenfalls mit einer Gruppe -CN oder einer Aminogruppe substituiert ist, eine 4'Aminophenylgruppe, oder eine Gruppe, die mit R₁ oder mit einem Kohlenstoffatom des Benzolrings, der die Gruppen R₃ und R₄ trägt, einen gegebenenfalls sauerstoffhaltigen und/oder stickstoffhaltigen Heterocyclus bildet, der mit einer C₁₋₄-Alkylgruppe substituiert sein kann,
- R₃ und R₄, die gleich oder verschieden sind, Wasserstoff, ein Halogenatom wie Brom, Chlor, Iod oder Fluor, eine C₁₋₄-Alkylgruppe oder C₁₋₄-Alkoxygruppe, eine Gruppe -CN,
- X ein Anion, das vorzugsweise unter Chlorid, Methylsulfat und Acetat ausgewählt ist,
- B eine Gruppe, die unter den folgenden Strukturen B1 bis B11 ausgewählt ist, 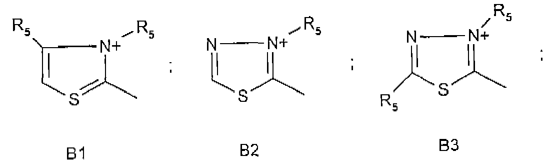 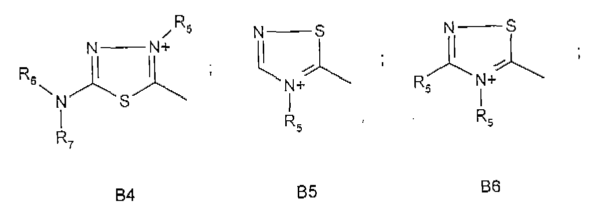 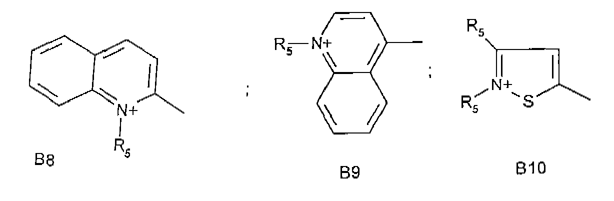 und 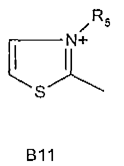 in denen R₅ eine C₁₋₄-Alkylgruppe bedeutet und R₆ und R₇, die gleich oder verschieden sind, Wasserstoff oder eine C₁₋₄-Alkylgruppe darstellen, wobei B für den Fall, daß R₁ und R₂ einen stickstoffhaltigen Heterocyclus bilden oder R₃ und R₉ eine C₁₋₄-Alkylgruppe oder eine C₁₋₄-Alkoxygruppe bedeuten oder R₂ eine 4'-Aminophenylgruppe ist, auch eine Gruppe der folgenden Struktur B12 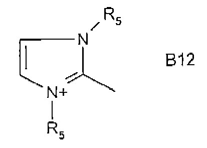 sein kann, in der R₅ die gleiche Bedeutung wie weiter oben bei den Strukturen B₁ bis B₁₁ angegeben hat, und
- mindestens ein Oxidationsmittel.

2. Zusammensetzung nach Anspruch 1, **dadurch gekennzeichnet, daß** die p-Phenylendiamine unter den Verbindungen der folgenden Formel (II) 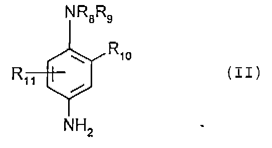 und deren Additionssalzen mit einer Säure ausgewählt sind, in der bedeuten:
R₈ Wasserstoff, C₁₋₄-Alkyl, C₁₋₄-Monohydroxyalkyl, C₂₋₄-Polyhydroxyalkyl, Phenyl, 4'-Aminophenyl oder C₁₋₄-Alkoxy-C₁₋₄-Alkyl; R₉ Wasserstoff, C₁₋₄-Alkyl, C₁₋₄-Monohydroxyalkyl oder C₂₋₄-Polyhydroxyalkyl;
R₁₀ Wasserstoff, ein Halogenatom wie Chlor, Brom, Iod oder Fluor, C₁₋₄-Alkyl, C₁₋₄-Monohydroxyalkyl, C₁₋₄-Hydroxyalkoxy, C₁₋₄-Mesylaminoalkoxy, C₁₋₄-Carbamoylaminoalkoxy oder C₁₋₄-Acetylaminoalkoxy;
R₁₁ Wasserstoff oder C₁₋₄-Alkyl.

3. Zusammensetzung nach Anspruch 2, **dadurch gekennzeichnet, daß** die p-Phenylendiamine der (II) ausgewählt sind unter p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-N,N-diethyl-3-methylanilin, N,N-Bis(β-hydroxyethyl)-p-phenylendiamin, 4-Amino-N,N-bis(β-hydroxyethyl)-anilin, 4-Amino-3-chlor-N,N-bis(β-hydroxyethyl)-anilin, 2-β-Hydroxyethyl-p-phenylendiamin, 2-Fluor-p-phenylendiamin, 2-Isopropyl-p-phenylendiamin, N-(β-Hydroxypropyl)-p-phenylendiamin, 2-Hydroxymethyl-p-phenylendiamin, N,N-Dimethyl-3-methyl-p-phenylendiamin, N,N-(Ethyl, β-Hydroxyethyl)-p-phenylendiamin, N-(β,γ-Dihydroxypropyl)-p-phenylendiamin, N-(4'-Aminophenyl)-p-phenylendiamin, N-Phenyl-p-phenylendiamin, 2-β-Hydroxyethyloxy-p-phenylendiamin, 2-β-Acetylaminoethyloxy-p-phenylendiamin und deren Additionssalze mit einer Säure.

4. Zusammensetzung nach Anspruch 1, **dadurch gekennzeichnet, daß** die Bisphenylalkylendiamine unter den Verbindungen der folgenden Formel (III) 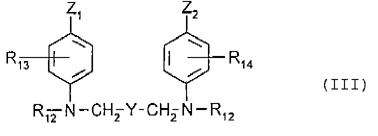 und deren Additionssalze mit einer Säure ausgewählt sind, in der bedeuten:
Z₁ und Z₂, die gleich oder verschieden sind, Hydroxy oder NHR₁₅, worin R₁₅ Wasserstoff oder C₁₋₄-Alkyl bedeutet,
R₁₂ Wasserstoff, C₁₋₄-Alkyl, C₁₋₄-Monohydroxyalkyl, C₂₋₄-Polyhydroxyalkyl oder C₁₋₄-Aminoalkyl, wobei die Aminoguppe substituiert sein kann,
R₁₃ und R₁₄, die gleich oder verschieden sind, Wasserstoff oder ein Halogenatom oder C₁₋₄-Alkyl,
Y eine Gruppe, die unter den folgenden Gruppen
-(CH₂)ₙ; -(CH₂)ₘ-O-(CH₂)ₘ -(CH₂)ₘ-CHOH-(CH₂)ₘ und 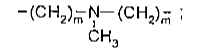 ausgewählt ist, worin n Null oder eine ganze Zahl von 1 bis einschließlich 8 und m Null oder eine ganze Zahl von 1 bis einschließlich 4 bedeutet.

5. Zusammensetzung nach Anspruch 4, **dadurch gekennzeichnet, daß** die Bisphenylalkylendiaminen der Formel (III) unter N,N'-Bis-(β-hydroxyethyl)-N,N'-bis(4'-aminophenyl)-1,3-diaminopropanol, N,N'-Bis(β-hydroxyethyl)-N,N'-bis(4'-aminophenyl)-ethylendiamin, N,N'-Bis(4-aminophenyl)-tetramethylendiamin, N,N'-Bis-(β-hydroxyethyl)-N,N'-bis(4-aminophenyl)-tetramethylendiamin, N,N'-Bis(4-methylaminophenyl)-tetramethylendiamin, N,N'-Bis-(ethyl)-N,N'-bis(9'-amino-3'-methylphenyl)-ethylendiamin und die Additionssalze dieser Verbindungen mit einer Säure.

6. Zusammensetzung nach einem der vorhergehenden Ansprüche, **dadurch gekennzeichnet, daß** die m-Phenylendiamine unter den Verbindungen der folgenden Formel (IV) 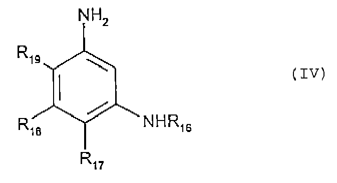 und deren Additionssalzen mit einer Säure ausgewählt sind, in der bedeuten:
- R₁₆ Wasserstoff, C₁₋₄-Alkyl, C₁₋₄-Monohydroxyalkyl, C₂₋₄-Polyhydroxyalkyl,
- R₁₇ und R₁₈, die gleich oder verschieden sind, Wasserstoff, C₁₋₄-Alkyl, C₁₋₄-Monohydroxalkoxy oder C₂₋₄-Polyhydroxyalkoxy,
- R₁₉ Wassserstoff, C₁₋₄-Alkoxy, C₁₋₄-Aminoalkoxy, C₁₋₄-Monohydroxyalkoxy, C₂₋₄-Polyhydroxyalkoxy oder 2,4-Diaminophenoxyalkoxy.

7. Zusammensetzung nach Anspruch 6, **dadurch gekennzeichnet, daß** die m-Phenylendiamine der Formel (IV) unter m-Phenylendiamin, 3,5-Diamino-1-ethyl-2-methoxybenzol, 3,5-Diamino-2-methoxy-1-methylbenzol, 2,4-Diamino-1-ethoxybenzol, 1,3-Bis(2,4-diaminophenoxy)propan, Bis(2,4-diaminophenoxy)methan, 1-(β-Aminoethyloxy)-2,4-diaminobenzol, 2-Amino-1-(β-hydroxyethyloxy)-4-methylaminobenzol, 2,4-Diamino-l-ethoxy-5-methylbenzol, 2,4-Diamino-5-(β-hydroxyethyloxy)-1-methylbenzol, 2,4-Diamino-1-(β,γ-Dihydroxypropyloxy)-benzol, 2,4-Diamino-1-(β-hydroxyethyloxy)-benzol, 2-Amino-4-N-(β-hydroxyethyl)-amino-1-methoxybenzol und deren Additionssalze mit einer Säure angegeben werden.

8. Zusammensetzung nach einem der vorhergehenden Ansprüche, **dadurch gekennzeichnet, daß** die direktziehenden kationischen Farbstoffe der Formel (I) unter den Verbindungen mit den folgenden Strukturen (I1) bis (I26) ausgewählt sind: 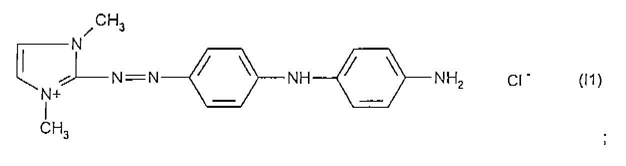 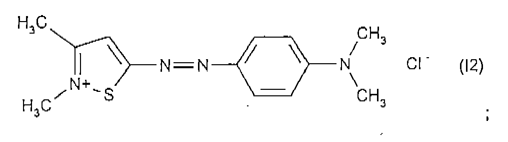 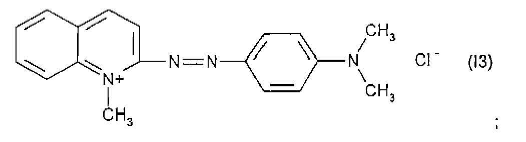 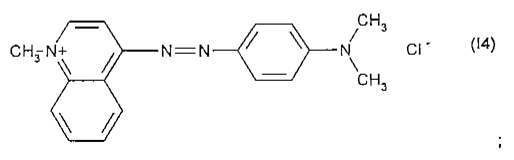 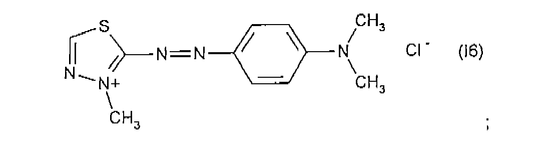 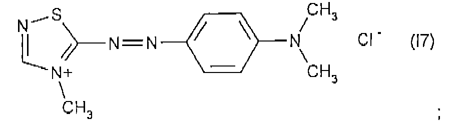 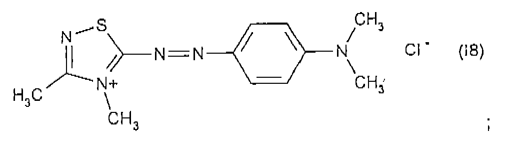 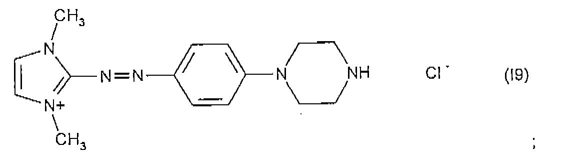 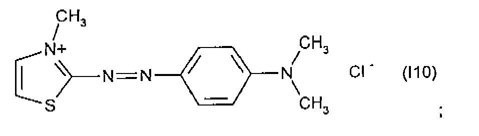 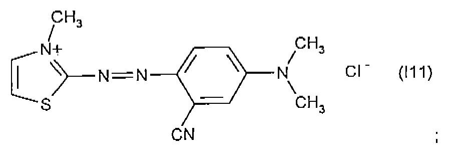 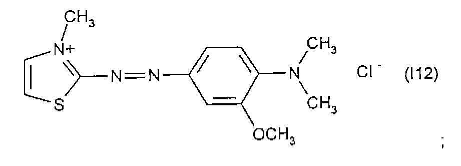 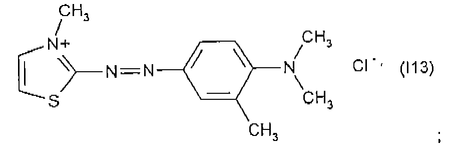 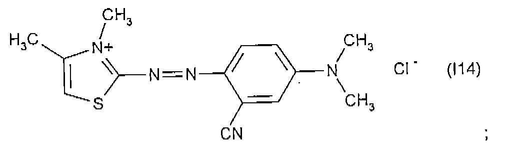 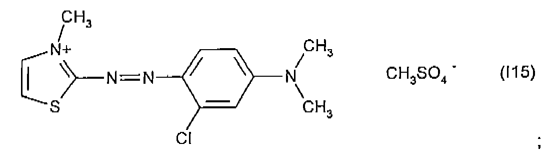 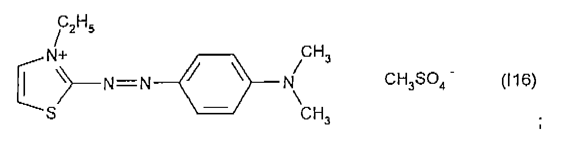 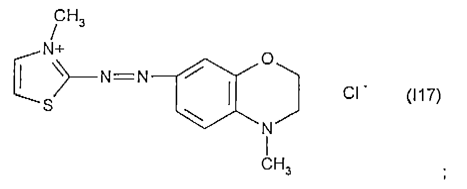 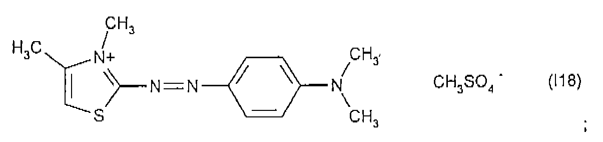 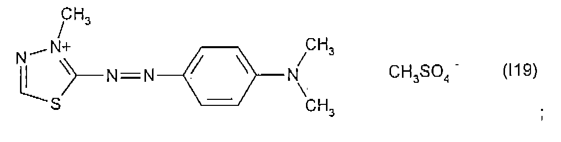 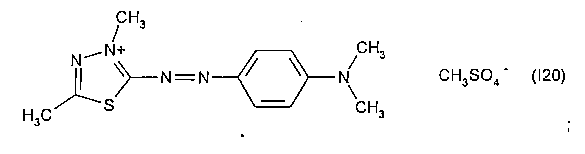 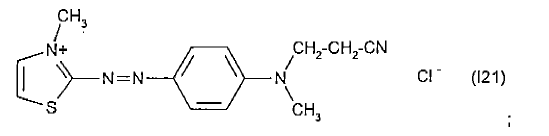 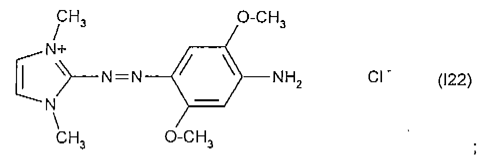 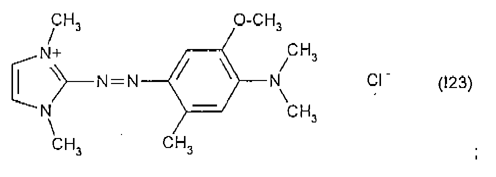 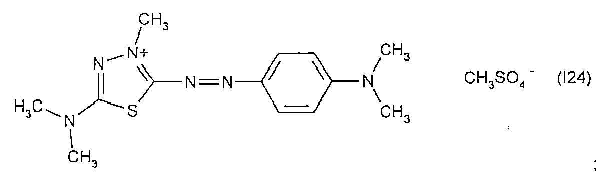 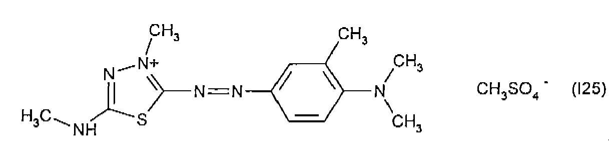 und

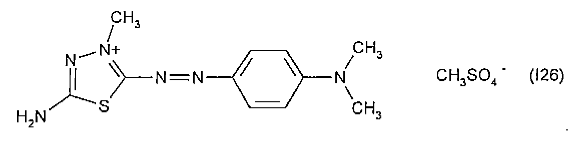

9. Zusammensetzung nach einem der vorhergehenden Ansprüche, **dadurch gekennzeichnet, daß** die Additionssalze mit einer Säure unter Hydrochloriden, Hydrobromiden, Sulfaten und Tartraten ausgewählt sind.

10. Zusammensetzung nach einem der vorhergehenden Ansprüche, **dadurch gekennzeichnet, daß** das Oxidationsmittel unter Wasserstoffperoxid, Harnstoffperoxid, Alkalimetallbromaten und Persalzen wie Perboraten und Persulfaten ausgewählt ist.

11. Zusammensetzung nach einem der vorhergehenden Ansprüche, **dadurch gekennzeichnet, daß** das oder die direktziehenden kationischen Farbstoffe 0,001 bis 10 Gew.-% des Gesamtgewichts der gebrauchsfertigen Färbemittelzusammensetzung ausmachen.

12. Zusammensetzung nach einem der vorhergehenden Ansprüche, **dadurch gekennzeichnet, daß** das oder die p-Phenylendiamine der Formel (II) und/oder das oder die Bisphenylalkylendiamine der Formel (III) 0,0001 bis 10 Gew.-% des Gesamtgewichts der gebrauchsfertigen Färbemittelzusammensetzung ausmachen.

13. Zusammensetzung nach einem der vorhergehenden Ansprüche, **dadurch gekennzeichnet, daß** das oder die m-Phenylendiamine der Formel (IV) 0,0001 bis 5 Gew.-% des Gesamtgewichts der gebrauchsfertigen Färbemittelzusammensetzung ausmachen.

14. Zusammensetzung nach einem der vorhergehenden Ansprüche, **dadurch gekennzeichnet, daß** der pH-Wert im Bereich von 5 bis 12 liegt.

15. Zusammensetzung nach einem der vorhergehenden Ansprüche, **dadurch gekennzeichnet, daß** das für die Färbung geeignete Medium aus Wasser oder einem Gemisch aus Wasser und mindestens einem organischen Lösungsmittel besteht.

16. Verfahren zur Färbung von Keratinfasern und insbesondere menschlichen Keratinfasern wie den Haaren, **dadurch gekennzeichnet, daß** auf diese Fasern mindestens eine wie in einem der Ansprüche 1 bis 15 beschriebene Färbemittelzusammensetzung aufgetragen wird.

17. Verfahren nach Anspruch 16, **dadurch gekennzeichnet, daß** es einen vorbereitenden Schritt umfaßt, der darin besteht, eine Zusammensetzung (A), die in einem für die Färbung geeigneten Medium mindestens eine Oxidationsbase, die unter p-Phenylendiaminen, Bisphenylalkylendiaminen und deren Additionssalzen mit einer Säure ausgewählt ist, mindestens einen Kuppler, der unter m-Phenylendiaminen und deren Additionssalzen mit einer Säure ausgewählt ist, und mindestens einen direktziehenden kationischen Farbstoff, der unter den Verbindungen der wie zuvor definierten Formel (I) ausgewählt ist, enthält, und eine Zusammensetzung (B), die in einem für die Färbung geeigneten Medium mindestens ein wie zuvor definiertes Oxidationsmittel enthält, getrennt voneinander zu lagern und zum Zeitpunkt ihrer Verwendung zu vermischen, bevor dieses Gemisch auf die Keratinfasern aufgetragen wird.

18. Färbeverfahren nach Anspruch 16, **dadurch gekennzeichnet, daß** es einen vorbereitenden Schritt umfaßt, der darin besteht, eine Zusammensetzung (A), die in einem für die Färbung geeigneten Medium mindestens eine Oxidationsbase, die unter p-Phenylendiaminen, Bisphenylalkylendiaminen und deren Additionssalzen mit einer Säure ausgewählt ist, und mindestens einen Kuppler, der unter m-Phenylendiaminen und deren Additionssalzen mit einer Säure ausgewählt ist, enthält, eine Zusammensetzung (A'), die in einem für die Färbung geeigneten Medium mindestens einen direktziehenden kationischen Farbstoff, der unter den Verbindungen der wie zuvor definierten Formel (I) ausgewählt ist, enthält, und eine Zusammensetzung (B), die in einem für die Färbung geeigneten Medium mindestens ein wie zuvor definiertes Oxidationsmittel enthält, getrennt voneinander zu lagern und diese Zusammensetzungen zum Zeitpunkt ihrer Verwendung zu vermischen, bevor dieses Gemisch auf die Keratinfasern aufgetragen wird.

19. Verfahren nach Anspruch 18, **dadurch gekennzeichnet, daß** die Zusammensetzung (A') in Form eines Pulvers vorliegt.

20. Färbevorrichtung mit mehreren Abteilungen oder Färbe-"Kit", **dadurch gekennzeichnet, daß** eine erste Abteilung die wie in Anspruch 17 definierte Zusammensetzung (A) und eine zweite Abteilung eine oxidierende Zusammensetzung (B) enthält.

21. Färbevorrichtung mit mehreren Abteilungen oder Färbe-"Kit", **dadurch gekennzeichnet, daß** eine erste Abteilung eine wie in Anspruch 18 definierte Zusammensetzung (A), eine zweite Abteilung eine wie in Anspruch 18 oder 19 definierte Zusammensetzung (A') und eine dritte Abteilung eine oxidierende Zusammensetzung (B) enthält.
